# EUROPEAN PATENT APPLICATION

(11) **EP 1 559 321 A1**
(43) Date of publication of application: **03.08.2005**
(21) Application number: 03807972.9
(22) Date of filing: 19.09.2003
(51) Int. Cl.: A01N 43/42, A01N 53/00, A61K 31/47, A61K 31/275, A61K 31/222, A61K 45/06, A61P 33/00, A61P 33/14, A61P 43/00

(54) **MIXED COMPOSITIONS FOR CONTROLLING PARASITIC INSECTS**

(30) Priority: 11.10.2002 JP 2002299653
(71) Applicant: Meiji Seika Kaisha, Ltd., Tokyo 104-8002 (JP)
(72) Inventor: OYAMA, Kazuhiko, Meiji Seika Kaisha, Ltd., Yokohama-shi, Kanagawa 222-8567 (JP)
(74) Representative: Warcoin, Jacques
(86) International application number: PCT/JP2003/012003
(87) International publication number: WO 2004/032629

(57) **Abstract**

The present invention relates to a mixture composition for controlling ectoparasites of mammals and birds, comprising as active ingredients a compound of formula (I) or a salt thereof and one or more compounds selected from the group consisting of pyrethroid insecticides. The composition according to the present invention has high control effect against resistant ectoparasites, can quickly act, and is safe. 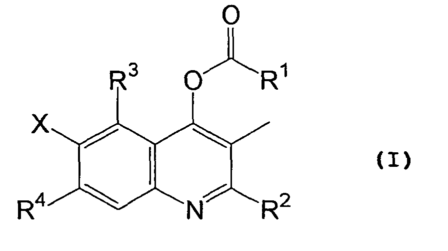 wherein R¹ represents optionally substituted alkyl; optionally substituted alkenyl; optionally substituted alkynyl; OR⁵ wherein R⁵ represents optionally substituted alkyl, optionally substituted alkenyl, or optionally substituted alkynyl; or SR⁵ wherein R⁵ is as defined above, R² represents optionally substituted alkyl, any one of R³ and R⁴ represents hydrogen and the other represents fluorine, chlorine, bromine, or CF₃, and X represents fluorine or chlorine.

## Description

### [BACKGROUND OF THE INVENTION]

### Field of the Invention

The present invention relates to a mixture composition for controlling ectoparasites which are harmful organisms parasitic on mammals and birds, comprising a halogen-substituted quinoline derivative and a pyrethroid insecticide.

### Related Art

Japanese Patent No. 2633377 discloses quinoline derivatives as insecticides for agricultural and horiticultural purposes. On the other hand, for example, "The Pesticide Manual," 12th edition, published by The British Crop Protection Council, 2000 describes that pyrethroid insecticides have control activity against ectoparasites. As a result of the use of these pyrethroid insecticides for many years, however, some ectoparasites have acquired drug resistance, and the control of the drug-resistant ectoparasites has become very difficult. Accordingly, the development of drugs which can control such drug-resistant ectoparasites has been desired.

Ectoparasites parasitic on mammals and birds induce symptoms such as malnutrition, weakness, weight reduction, and decrease in an egg laying rate of host animals through hematophagia. A large number of control agents have hitherto been developed for these ectoparasites. However, quick-acting and safe drugs, which can more rapidly reduce stress on the host animals, have been desired.

### [SUMMARY OF THE INVENTION]

The present inventors have now found that a mixture composition comprising a derivative with a halogen substituent at a specific position of 4-acyloxyquinoline and at least one compound used as pyrethroid insecticides has significant synergistic effect on control effect against ectoparasites of mammals and birds, as compared with the case where the halogen-substituted quinoline derivative and the compound used as pyrethroid insecticide each are solely used. The present invention has been made based on such finding.

Accordingly, an object of the present invention is to provide a chemical agent for controlling ectoparasites of mammals and birds, which has high control effect against resistant ectoparasites, can quickly act, and is safe.

According to the present invention, there are provided a mixture composition for controlling ectoparasites of mammals and birds, comprising as active ingredients:
(a) a compound of formula (I) or a salt thereof: 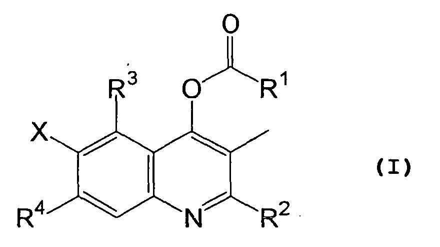 wherein
   R¹ represents alkyl having 1 to 6 carbon atoms optionally substituted by a halogen atom or cyano; alkenyl having 2 to 6 carbon atoms optionally substituted by a halogen atom or cyano; alkynyl having 2 to 6 carbon atoms optionally substituted by a halogen atom or cyano; group -O-R⁵ wherein R⁵ represents alkyl having 1 to 6 carbon atoms optionally substituted by a halogen atom or cyano, alkenyl having 2 to 6 carbon atoms optionally substituted by a halogen atom or cyano, or alkynyl having 2 to 6 carbon atoms optionally substituted by a halogen atom or cyano; or group -S-R⁵ wherein R⁵ is as defined above,
   R² represents alkyl having 1 to 4 carbon atoms optionally substituted by a halogen atom,
   any one of R³ and R⁴ represents a hydrogen atom and the other represents a fluorine atom, a chlorine atom, a bromine atom, or trifluoromethyl (-CF₃), and
   X represents a fluorine atom or a chlorine atom, and
(b) one or more compounds selected from the group consisting of pyrethroid insecticides.

According to the present invention, there is provided a method for controlling an ectoparasite of mammals and birds, comprising the step of administering an effective amount of the mixture composition for controlling ectoparasites according to the present invention to a mammal or a bird of interest.

### [DETAILED DESCRIPTION OF THE INVENTION]

### Mixture composition for controlling ectoparasites

As descried above, the mixture composition for controlling ectoparasites of mammals and birds according to the present invention comprises as active ingredients a compound of formula (I) or a salt thereof and one or more compounds selected from the group consisting of pyrethroid insecticides.

In the present invention, "comprising as an active ingredient" means that a carrier suitable for desired formulation may of course be incorporated and, in addition, other chemical agents usable in combination with the compounds of the present invention may be incorporated.

### Compounds of formula (I)

The term "halogen atom" as used herein means a fluorine, chlorine, bromine, or iodine atom, preferably a fluorine or chlorine atom.

The term "alkyl" as used herein means straight chain alkyl, branched chain alkyl, or cyclo (cyclic) alkyl. Examples of alkyl include: straight chain alkyl such as methyl, ethyl, propyl, butyl, pentyl, and hexyl; branched chain alkyl such as isopropyl, isobutyl, s-butyl, t-butyl, neopentyl, isopentyl, and isohexyl; and cycloalkyl such as cyclopropyl, 1-methylcyclopropyl, cyclopropylmethyl, cyclobutyl, cyclopentyl, and cyclohexyl.

The term "alkenyl" as used herein means straight chain alkenyl, branched chain alkenyl, or cycloalkenyl. Examples of alkenyl include vinyl, allyl, propenyl, isopropenyl, butenyl, 2-butenyl, 3-butenyl, 2-methyl-1-propenyl, 2-methylallyl, pentenyl, 2-pentenyl, 3-pentenyl, 4-pentenyl, cyclopentenyl, hexenyl, 2-hexenyl, 3-hexenyl, 4-hexenyl, 5-hexenyl, and cyclohexenyl.

The term "alkynyl" as used herein means straight chain alkynyl, branched chain alkynyl, or cycloalkynyl. Examples of alkynyl include ethynyl, propynyl, 2-propynyl, butynyl, 2-butynyl, 3-butynyl, pentynyl, 2-pentynyl, 3-pentynyl, 4-pentynyl, hexynyl, 2-hexynyl, 3-hexynyl, 4-hexynyl, and 5-hexynyl.

Alkyl having 1 to 6 carbon atoms is preferably alkyl having 1 to 4 carbon atoms.

Alkenyl having 2 to 6 carbon atoms is preferably alkenyl having 2 to 4 carbon atoms.

Alkynyl having 2 to 6 carbon atoms is preferably alkynyl having 2 to 4 carbon atoms.

In the present invention, examples of preferred compounds of formula (I) are the compounds of formula (I) wherein any one of R³ and R⁴ represents a hydrogen atom and the other represents a fluorine or chlorine atom.

In a preferred embodiment of the present invention, the compound of formula (I) may be preferably compounds of formula (Ia) : 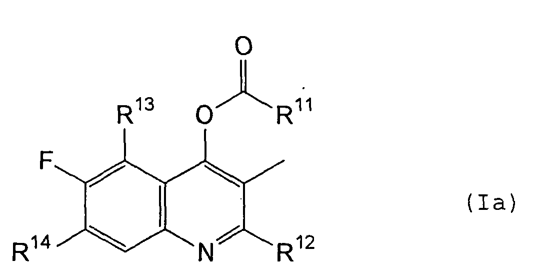 wherein
R¹¹ represents alkyl having 1 to 4 carbon atoms optionally substituted by a halogen atom or alkoxy having 1 to 4 carbon atoms optionally substituted by a halogen atom;
R¹² represents alkyl having 1 to 4 carbon atoms optionally substituted by a halogen atom; and
any one of R¹³ and R¹⁴ represents a hydrogen atom and the other represents a fluorine or chlorine atom.

In the present invention, examples of preferred compounds of formula (I) are compounds 1 to 81 described in working examples.

According to a further preferred aspect of the present invention, examples of the compounds of formula (I) may be selected from the group consisting of:
2-ethyl-3-methyl-4-cyclopropanecarbonyloxy-5,6-difluoroquinoline (Compound 2);
2-ethyl-3-methyl-4-acetyloxy-5-chloro-6-fluoroquinoline (Compound 19);
2-ethyl-3-methyl-4-methoxycarbonyloxy-5,6-difluoroquinoline (Compound 24);
2,3-dimethyl-4-methoxycarbonyloxy-5,6-difluoroquinoline (Compound 25);
2-ethyl-3-methyl-4-methanethiolcarbonyloxy-5,6-difluoroquinoline (Compound 39);
2-ethyl-3-methyl-4-propargyloxycarbonyloxy-5,6-difluoroquinoline (Compound 41);
2,3-dimethyl-4-acetoxy-5,6-difluoroquinoline (Compound 44);
2,3-dimethyl-4-methanethiolcarbonyloxy-5,6-difluoroquinoline (Compound 46);
2,3-dimethyl-4-propargyloxycarbonyloxy-5,6-difluoroquinoline (Compound 50);
2,3-dimethyl-4-(3-butynyl)oxycarbonyloxy-5,6-difluoroquinoline (Compound 52);
2-ethyl-3-methyl-4-(3-butynyl)-oxycarbonyloxy-5,6-difluoroquinoline (Compound 56);
2,3-dimethyl-4-allyloxycarbonyloxy-5,6-difluoroquinoline (Compound 57);
2,3-dimethyl-4-acetoxy-5,6-dichloroquinoline (Compound 65); and
2,3-dimethyl-4-methoxycarbonyloxy-5,6-dichloroquinoline (Compound 67).

The compounds of formula (I) may form salts, and examples thereof include hydrochloric acid salts, nitric acid salts, phosphoric acid salts, and acetic acid salts.

### Production process of compounds of formula (I)

The compounds of formula (I) may be produced, for example, according to a method shown in scheme 1. For the production of these compounds, reference may be made to Japanese Patent Laid-Open No. 128355/1991. 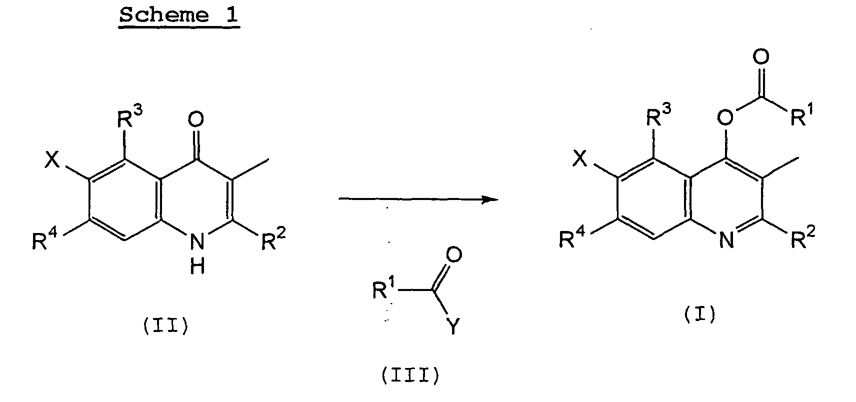 wherein Y represents a chlorine atom or hydroxyl; and R¹, R², R³, R⁴ and X are as defined in formula (I).

A compound of formula (I) can be synthesized by reacting a compound of formula (II) with a reagent represented by formula (III) in the absence of a solvent or in the presence of a suitable solvent. When Y represents a chlorine atom, the compound of formula (I) can be synthesized by carrying out the reaction in the presence of a suitable base, for example, an organic amine, such as triethylamine or pyridine, or an inorganic alkali, such as sodium carbonate, potassium carbonate, or sodium hydride. When Y represents hydroxyl, the compound of formula (I) can be synthesized by carrying out the reaction in the presence of a suitable condensing agent, for example, dicyclohexylcarbodiimide (DCC), 1-hydroxybenzotriazole (HOBt), or 2-chloro-1,3-dimethylimidazolinium chloride (DMC).

The reagent represented by formula (III) is preferably used in an amount in the range of 1 to 50 equivalents, preferably in the range of 1 to 10 equivalents, based on the compound of formula (II). Solvents usable herein are organic solvents inert to the reaction, for example, dimethylformamide, tetrahydrofuran, dimethyl sulfoxide, and dichloromethane. The reaction may be carried out at a temperature in the range of 0°C to 140°C.

The compound of formula (II) as a start compound in scheme 1 may be produced according to a method shown in scheme 2. In the production of the compound of formula (II), reference may be made to J. Am. Chem. Soc. 70, 2402 (1948), Tetrahedron Lett. 27, 5323 (1986).

Specifically, the compound of formula (II) may be produced by imidating a halogen-substituted aniline, which may be synthesized by a conventional method or is commercially available, with a β-ketoester in the absence or presence of an acid catalyst in a suitable solvent, for example, benzene, toluene, or xylene, and further cyclizing the imide compound in the absence of a solvent or in the presence of a high-boiling solvent such as diphenyl ether or cyclizing the imide compound in a solvent such as toluene or xylene in the presence of an acid catalyst. Acid catalysts usable in the imidation and the cyclization include trifluoroboron etherate, p-toluenesulfonic acid, and polyphosphoric acid. The imidation and the cyclization may be carried out, if appropriate, in two steps in respective different solvents or the same solvent. 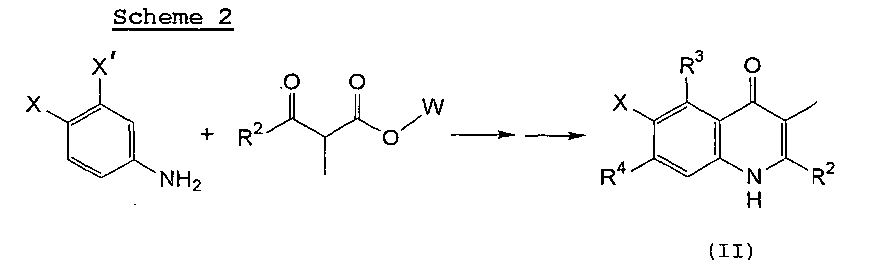 wherein R², R³, R⁴, and X are as defined in formula (I); X' represents a fluorine atom, a chlorine atom, a bromine atom, or -CF₃; and W represents methyl or ethyl.

### Pyrethroid insecticides

The pyrethroid insecticides usable in the present invention may be any compound which is known as pyrethroid insecticide in the art.

These pyrethroid insecticides have already been described as insecticides, for example, in "The Pesticide Manual," 12th edition, published by The British Crop Protection Council, 2000.

Specific examples of pyrethroid insecticides usable in the mixture composition for controlling ectoparasites according to the present invention include compounds such as pyrethrin, allethrin, prallethrin, proparthrin, furamethrin, pyresmethrin, resmethrin, cismethrin, bioresmethrin, kadethrin, imiprothrin, phenothrin, permethrin, transfluthrin, fenfruthrin, tefluthrin, bifenthrin, fenpropathrin, cyfenothrin, cyhalothrin, cypermethrin, cyfluthrin, deltamethrin, tralomethrin, acrinathrin, flumethrin, empenthrin, phthalthrin, flucythrinate, fenvalerate, fluvalinate, cycloprothrin, ethofenprox, halfenprox, and silafluofen.

According to a preferred aspect of the present invention, the pyrethroid insecticide is selected from the group consisting of flumethrin, permethrin, and cyfluthrin.

### Other ingredients

In the control of ectoparasites of mammals and birds, the mixture composition for controlling ectoparasites according to the present invention, which comprises an effective amount of one or more compounds of formula (I) and one or more compounds selected from the group consisting of pyrethroid insecticides, together with preparation additives, can be administered by oral administration; parenteral administration such as injection such as intramuscular, subcutaneous, intraveneous, or intraperitoneal injection; percutaneous administration such as dipping, spray, bathing, washing, pouring-on, spotting-on, and dusting; or nasal administration. Alternatively, the mixture composition according to the present invention may be administered by shaped products using strips, plates, bands, collars, ear marks, limb bands, labeling devices or the like.

When the mixture composition for controlling ectoparasites according to the present invention is actually applied, a composition consisting of the above active ingredients only as such may be used. Alternatively, the composition may be mixed, for example, with suitable carriers or adjuvants for formulations, to prepare any suitable formulation. Accordingly, for the administration, the mixture composition for controlling ectoparasites according to the present invention may be formulated into preparations of suitable dosage forms according to the administration routes.

Suitable dosage forms which may be formulated include: solid preparations such as powders, granules, wettable powders, pellets, tablets, boluses, capsules, and shaped products containing active compounds; liquid preparations for injections, oral liquid preparations, and liquid preparations used on skins or in coeloms; solution preparations such as pour-on or spot-on preparations, floable preparations, and emulsions; and semi-solid preparations such as ointments and gels.

Solid preparations may be mainly used for oral administration or alternatively may be diluted with water or the like before percutaneous administration or environmental treatment. Solid preparations may be prepared by mixing the active compound with a suitable excipient, if necessary, after the addition of adjuvants and bringing the mixture into a desired shape. Suitable excipients include, for example, inorganic materials such as carbonates, hydrogencarbonates, phosphates, ammonium oxide, silica (silicon dioxide),kaolin, and clay and organic materials such as saccharides, cellulose, ground grains, and starch.

Liquid preparations for injections may be intraveneously, intramuscularly, or subcutaneously administered. Liquid preparations for injections may be prepared by dissolving the active compound in a suitable solvent and optionally adding additives, such as a solubilizer, an acid, a base, a salt for buffering, an antioxidant, antifoamer and a protective agent, to the solution. Suitable solvents include water, ethanol, butanol, 2-propanol, benzyl alcohol, glycerin, propylene glycol, polyethylene glycol, N-methylpyrrolidone, and mixtures of these solvents, physiologically acceptable vegetable oils, and synthetic oils for injections. Solubilizers include polyvinylpyrrolidone, Polyoxyethylene lauryl ether, Sodium dioctylsulfosuccinate, polyoxyethylated castor oils, and polyoxyethylated sorbitan esters. Protective agents include benzyl alcohol, trichlorobutanol, p-hydroxybenzoic esters, and n-butanol. Antifoamers include emulsion-type silicone.

Oral liquid preparations may be administered either directly or after dilution and may be prepared in the same manner as in the preparation of liquid preparations for injections.

Floable preparations, emulsions and the like may be administered percutaneously or by the environmental treatment either directly or after dilution.

Liquid preparations for use on the skin may be administered by pouring-on or spotting-on, spreading, rubbing-in, spray, or application, or immersion (dipping, bathing, or washing) for coating. They may be prepared in the same manner as in the preparation of liquid preparations for injections.

Pour-on and spot-on preparations may be dropped or sprayed on a limited site of the skin to allow the active compound to penetrate into the skin and thus to cause systemic action of the active compound. Pour-on and spot-on preparations may be prepared by dissolving, suspending, or emulsifying the active ingredient in a suitable solvent or solvent mixture compatible with the skin. If necessary, adjuvants such as surfactants, colorants, absorbefacients, antioxidants, photostabilizers, and adhesives may be added to the pour-on and spot-on preparations.

Suitable solvents include water, alkanols, glycols, polyethylene glycol, 2-propanol, isopropanol, polypropylene glycol, dipropylene glycol, hexylene glycol, glycerin, benzyl alcohol, phenylethanol, phenoxyethanol, ethyl acetate, butyl acetate, benzyl benzoate, propylene glycol monomethyl ether, dipropylene glycol monomethyl ether, diethylene glycol monobutyl ether, alkylaryl polyglycol ether, acetone, methyl ethyl ketone, aromatic and/or aliphatic hydrocarbons, vegetable or synthetic oils, liquid paraffin, light liquid paraffin, silicone, DMF, dimethylacetamide, N-methylpyrrolidone, and 2,2-dimethyl-4-oxy-methylene-1,3-dioxolane. Absorbefacients include dimethyl sulfoxide (DMSO), isopropyl myristate, dipropylene glycol pelargonate, silicone oils, aliphatic esters, triglycerides, and fatty alcohols. Antioxidants include bisulfites, metabisulfites, ascorbic acid, butylhydroxytoluene, butylhydroxyanisole, and tocopherol.

Emulsions may be administered orally, percutaneously, or by injection. Emulsions may be prepared by dissolving the active ingredient in a hydrophobic phase or a hydrophilic phase and homogenizing the solution in other phase with the aid of a suitable emulsifier, if necessary, together with adjuvants such as a colorant, an absorbefacient, a protective agent, an antioxidant, a light-screening agent, and a thickening agent.

Hydrophobic phases (oils) include paraffin oils, silicone oils, sesame oils, almond oils, castor oils, olive oils, medium chain triglyceride, synthetic triglyceride, ethyl stearate, di-n-butyryl adipate, hexyl laurylate, dipropylene glycol pelargonate, esters of branched short-chain fatty acids with saturated fatty acids having 16 to 18 carbon atoms, isopropyl myristate, isopropyl palmitate, esters of caprylic acid/capric acid with saturated fatty alcohols having 12 to 18 carbon atoms, isopropyl stearate, oleyl oleate, decyl oleate, ethyl oleate, ethyl lactate, waxy fatty esters, dibutyl phthalate, diisopropyl adipate, isotridecyl alcohol, 2-octyldodecanol, cetylstearyl alcohol, and oleyl alcohol.

Hydrophilic phases include water, propylene glycol, glycerin, and sorbitol.

Emulsifiers include: nonionic surfactants such as polyoxyethylated castor oils, polyoxyethylated monoolefinic acid sorbitan, sorbitan monostearate, glycerin monostearate, polyoxyethyl stearate, and alkylphenol polyglycol ether; amphoteric surfactants such as disodium N-lauryl-β-iminodipropionate and lecithin; anionic surfactants such as sodium laurylsulfate, sodium diisobutylnaphthalenesulfonate, calcium n-dodecylbenzenesulfonate, sodium salt of naphthalenesulfonic acid formalin condensate, fatty alcohol sulfuric acid ether, and monoethanolamine salt of mono-/dialkylpolyglycol orthophosphate; and cationic surfactants such as cetyltrimethylammonium chloride.

Other adjuvants include carboxymethylcellulose, methylcellulose, polyacrylate, alginate, gelatin, gum arabic, polyvinylpyrrolidone, polyvinyl alcohol, methyl vinyl ether, maleic anhydride copolymers, polyethylene glycol, wax, and colloidal silica.

Semi-solid preparations may be administered by coating or spreading on the skin or by introduction into the coelom. The gel may be prepared by adding a thickener, suitable for forming a transparent material having ointment-like viscous properties, to the solution prepared above in connection with liquid preparations for injections.

The above other ingredients may be used solely or in a combination of two or more of them selected from the same group or different groups.

In preparing these preparations, other insecticides, for example, organophosphorus insecticides, carbamate insecticides, or pyrethroid insecticides may be mixed.

Further, in preparing these preparations, synergists such as piperonyl butoxide may be mixed. Furthermore, in preparing these preparations, other insecticides for controlling ectoparasites, endoparasite control agents such as anthelmintics, or antimicrobial agents, etc. may be mixed.

In the mixture composition for controlling ectoparasites according to the present invention, the mixing proportion of the compound of formula (I) or a salt thereof and one or more compounds selected from the group consisting of pyrethroid insecticides may be such that the total amount of them (the total amount of the active ingredients) is 0.1 to 99 parts by weight, preferably 1 to 50 parts by weight, based on 100 parts by weight of the mixture composition for controlling ectoparasites.

The total amount of the active ingredients may be properly selected by taking into consideration formulation, aministration method, service environment, and other conditions of the mixture composition for controlling ectoparasites. For example, when the mixture composition for controlling ectoparasites is in a wettable powder form, the total amount of the active ingredients is 0.1 to 99% by weight, preferably 0.5 to 50% by weight. On the other hand, when the mixture composition for controlling ectoparasites is in a dust form, the total amount of the active ingredients is 0.1 to 99% by weight, preferably 0.1 to 30% by weight.

In the mixture composition for controlling ectoparasites according to the present invention, the mixing ratio between the compound of formula (I) or a salt thereof and one or more compounds selected from the group consisting of pyrethroid insecticides is in the range of 1 : 99 to 99 : 1, preferably in the range of 10 : 90 to 90 : 10.

When the mixture composition for controlling ectoparasites according to the present invention is used, the mixture composition for controlling ectoparasites as such may be used directly. If necessary, the mixture composition for controlling ectoparasites may be diluted with a diluting liquid, such as water, followed by orally or parenterally administer to a mammal or a bird of interest.

### Use of composition

As demonstrated in Test Example 1, the mixture composition for controlling ectoparasites of mammals and birds according to the present invention had excellent control activity against ectoparasites parasitic on homotermic animals. Therefore, the mixture composition for controlling ectoparasites according to the present invention can be used for controlling ectoparasites parasitic, for example, on mammals, such as humans, cattle, horses, pigs, sheep, goats, camels, donkeys, dogs, cats, rabbits, monkeys, guinea pigs, and hamsters, and birds such as chickens, ducks, gooses, and turkeys.

Such ectoparasites include: Anoplura such as Haematopinus spp., Linognathus spp., Pediculus spp., and Phtirus spp.; Mallophaga such as Menopon spp., Eomenacanthus, Felicola spp., and Damalina spp.; Siphonaptera such as Ctenocephalides spp., Echidnophaga spp., and Pulex spp.; parasitic ticks such as Rhipicephalus spp., Haemaphysalis longicornis, Boophilus spp., Amblyomma spp., Dermacentor spp., Ixodes spp., Argas spp., and Ornithonyssus sylviarum; Mesostigmata such as Dermanyssus spp., Psoroptes spp., Sarcoptes spp., Notoedres spp., and Knemidocoptes spp.; and Diptera such as Chrysops spp., Tabanus spp., Stomoxys spp., Lucilia spp., Calliphora spp., Chrysomia spp., Sarcophaga spp., Gastrophilus spp., Oestrus spp., and Hippobosca spp.

These ectoparasites are sometimes parasitic in the body of mammals and birds. The mixture composition for controlling ectoparasites according to the present invention may also be used for controlling parasites parasitic in the body.

In the present invention, "controlling" ectoparasites embraces exterminating ectoparasites which are parasites on mammals and birds, and preventing ectoparasites from prasitizing mammals and birds.

According to another aspect of the present invention, there is provided a method for controlling an ectoparasite of mammals and birds, comprising the step of administering an effective amount of the mixture composition according to the present invention to a mammal or a bird of interest. The term "a mammal or a bird of interest" herein refers to a mammal or a bird which should control ectoparasites, i.e., a mammal or a bird which is parasitic on or in, or may be parasitic on or in.

The amount of the mixture composition for controlling ectoparasites according to the present invention used may be properly varied depending upon service environment, condition of object mammals or birds, mixing ratio of active ingredients, formulations, administration method, and target ectoparasite to be controlled.

In general, the amount of the mixture composition for controlling ectoparasites used is 0.0001 to 10 g, preferably 0.001 to 1 g, per 1 kg of weight of object mammals or birds in terms of the total amount of the active ingredients.

In general, the mixture composition for controlling ectoparasites according to the present invention is previously formed into a desired formulation in the above-described manner. Alternatively, a method may be adopted wherein a formulation comprising the active ingredients in the composition, that is, one of the compounds of formula (I) or a salt thereof and one or more compounds selected from the group consisting of pyrethroid insecticides, and a formulation comprising the other active ingredient are previously prepared and, in use, these formulations are mixed together on site.

Thus, according to another aspect of the present invention, there is provided a combination comprising a compound of formula (I) or a salt thereof and one or more compounds selected from the group consisting of pyrethroid insecticides.

In a preferred embodiment, in the above combination, the compound of formula (I) or a salt thereof is provided as a first composition comprising the compound of formula (I) or a salt thereof as an active ingredient, and the one or more compounds selected from the group consisting of pyrethroid insecticides is provided as a second composition comprising the one or more compounds as an active ingredient. In this case, as with the above-described mixture composition for controlling ectoparasites, the first composition and the second composition may be in any formulation form which has been prepared by additionally using suitable carrier or adjuvant. This combination may be provided in such a form as a chemical set.

According to another aspect of the present invention, there is provided use of the above-described combination, for the production of a mixture composition for controlling ectoparasites of mammals and birds. The mixture composition for controlling ectoparasites of mammals and birds may express parasiticide in different words.

According to still another aspect of the present invention, there is provided a method for controlling an ectoparasite of mammals and birds, comprising the step of administering simultaneously or separately to a mammal or a bird of interest:
(a) a compound of formula (I) or a salt thereof;
(b) one or more compounds selected from the group consisting of pyrethroid insecticides.

In this method, administering the above ingredients (a) and (b) "simultaneously" embraces that the compound of formula (I) or a salt thereof and one or more compounds selected from the group consisting of pyrethroid insecticides may be mixed together before administration of the mixture to mammals and birds of interest. Administering the above ingredients (a) and (b) "separately" from each other embraces that the compound of formula (I) or a salt thereof is applied before the other ingredient, and that the compound of formula (I) or a salt thereof is applied after the other ingredient, without pre-mixing those ingredients.

In a further preferred embodiment of the present invention, there is provided a method for controlling an ectoparasite of mammals and birds, comprising the step of administering:
(A) a first composition comprising a compound of formula (I) or a salt thereof as an active ingredient, and
(B) a second composition comprising one or more compounds selected from the group consisting of pyrethroid insecticides as an active ingredient, to a mammal or a bird of interest.

### [EXAMPLES]

The present invention is further illustrated by the following Examples that are not intended as a limitation of the invention.

### Example 1: 2-Ethyl-3-methyl-4-cyclopropanecarbonyloxy-6,7-difluoroquinoline (compound 1) and 2-ethyl-3-methyl-4-cyclopropanecarbonyloxy-5,6-difluoroquinoline (compound 2)

3,4-Difluoroaniline (3.18 g) and 3.9 g of ethyl-2-methylpropionyl acetate were refluxed in toluene (50 ml) in the presence of 0.3 ml of boron trifluoride etherate for 3 hr. The reaction mixture obtained was washed with a saturated sodium hydrogencarbonate solution and saturated brine and was dried over anhydrous sodium sulfate, and the solvent was then removed by evaporation. The resultant intermediate was refluxed in diphenyl ether for 30 min and was allowed to stand for cooling. The precipitate was then collected by filtration under the reduced pressure to give 1.9 g of a mixture of 2-ethyl-3-methyl-4-hydroxy-6,7-difluoroquinoline with 2-ethyl-3-methyl-4-hydroxy-5,6-difluoroquinoline. A 60% sodium hydride (20 mg) was suspended in 2 ml of dimethyl formamide. Separately, the mixture (100 mg) of 2-ethyl-3-methyl-4-hydroxy-6,7-difluoroquinoline and 2-ethyl-3-methyl-4-hydroxy-5,6-difluoroquinoline (starting material 1) was suspended in 2 ml of dimethyl formamide, and the suspension of the starting material 1 was added dropwise to the above suspension of sodium hydride under ice cooling. The mixture was stirred for one hr, and 70 µl of cyclopropanecarbonyl chloride (starting material 2) was then added thereto. The mixture was stirred at room temperature for 3 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate, and the solvent was then removed by evaporation under the reduced pressure. The crude product thus obtained was purified on Wako Gel C-200 (manufactured by Wako Pure Chemical Industries, Ltd.)(100 ml; elution solvent = n-hexane-ethyl acetate (10 : 1)) to give 78.8 mg of 2-ethyl-3-methyl-4-cyclopropanecarbonyloxy-6,7-difluoroquinoline (compound 1) and 17.8 mg of 2-ethyl-3-methyl-4-cyclopropanecarbonyloxy-5,6-difluoroquinoline (compound 2). ¹H-NMR data were shown in Table 3.

### Alternative to Example 1

### Synthesis of starting material 1 (mixture of 2-ethyl-3-methyl-4-hydroxy-6,7-difluoroquinoline with 2-ethyl-3-methyl-4-hydroxy-5,6-difluoroquinoline)

3,4-Difluoroaniline (1.0 g) and 1.12 g of methyl-2-methylpropionyl acetate were stirred in 5 ml of xylene in the presence of 0.3 ml of boron trifluoride etherate at 140°C for 3 hr. The reaction solution obtained was allowed to stand for cooling, and the precipitate was then collected by filtration to give 603.7 mg of a mixture of 2-ethyl-3-methyl-4-hydroxy-6,7-difluoroquinoline with 2-ethyl-3-methyl-4-hydroxy-5,6-difluoroquinoline.

### Example 2: 2-Ethyl-3-methyl-4-acetyloxy-6,7-difluoroquinoline (compound 3)

2-Ethyl-3-methyl-4-cyclopropanecarbonyloxy-6,7-difluoroquinoline (5.5 g) prepared as described in Example 1 was dissolved in 50 ml of methanol. A solution of 2.5 g of sodium hydroxide in 50 ml of water was added to this solution, and the mixture was stirred at 50°C for 3 hr. The reaction solution obtained was allowed to stand for cooling and was then poured into 50 ml of water. The mixture was neutralized with 1N hydrochloric acid, and the precipitate was then collected by filtration to give 5.1 g of 2-ethyl-3-methyl-4-hydroxy-6,7-difluoroquinoline. A 60% sodium hydride (96 mg) was suspended in 20 ml of tetrahydrofuran. Separately, 446 mg of the 2-ethyl-3-methyl-4-hydroxy-6,7-difluoroquinoline (starting material 1) was suspended in 10 ml of tetrahydrofuran, and this suspension of starting material 1 was added dropwise to the above suspension of sodium hydride under ice cooling. The mixture was stirred for 1 hr, and 188.4 mg of acetyl chloride (starting material 2) was then added thereto. The mixture was stirred at room temperature for 15 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate, and the solvent was then removed by evaporation under the reduced pressure. The crude product thus obtained was purified on Wako Gel C-200 (100 ml; elution solvent = n-hexane-ethyl acetate (10 : 1)) to give 168.6 mg of 2-ethyl-3-methyl-4-acetyloxy-6,7-difluoroquinoline (compound 3). ¹H-NMR data were shown in Table 3.

### Example 3: 2-Ethyl-3-methyl-4-acetyloxy-5,6-difluoroquinoline (compound 12)

2-Ethyl-3-methyl-4-cyclopropanecarbonyloxy-5,6-difluoroquinoline (1 g) prepared as described in Example 1 was dissolved in 10 ml of methanol to prepare a solution. A solution of 0.5 g of sodium hydroxide in 10 ml of water was added to this solution, and the mixture was stirred at 50°C for 3 hr. The reaction solution obtained was allowed to stand for cooling and was then poured into 50 ml of water, and the mixture was neutralized with 1 N hydrochloric acid. The precipitate was then collected by filtration to give 700 mg of 2-ethyl-3-methyl-4-hydroxy-5,6-difluoroquinoline. A 60% sodium hydride (96 mg) was suspended in 20 ml of tetrahydrofuran. Separately, the 2-ethyl-3-methyl-4-hydroxy-5,6-difluoroquinoline (starting material 1) (446 mg) was suspended in 10 ml of tetrahydrofuran, and this suspension of starting material 1 was added dropwise to the above suspension of sodium hydride under ice cooling. The mixture was stirred for one hr, 200 mg of acetyl chloride (starting material 2) was then added thereto, and the mixture was stirred at room temperature for 4.5 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate. The solvent was then removed by evaporation under the reduced pressure. The crude product thus obtained was purified on Wako Gel C-200 (100 ml; elution solvent = n-hexane-ethyl acetate (10 : 1)) to give 424 mg of 2-ethyl-3-methyl-4-acetyloxy-5,6-difluoroquinoline (compound 12). ¹H-NMR data were shown in Table 3.

### Example 4: 2,3-Dimethyl-4-cyclopropanecarbonyloxy-6,7-difluoroquinoline (compound 15) and 2,3-dimethyl-4-cyclopropanecarbonyloxy-5,6-difluoroquinoline (compound 16)

3,4-Difluoroaniline (5.16 g) and 5.76 g of ethyl-2-methyl acetoacetate were refluxed in toluene (80 ml) in the presence of 0.3 ml of boron trifluoride etherate for 3 hr. The reaction mixture obtained was washed with a saturated sodium hydrogencarbonate solution and saturated brine and was dried over anhydrous sodium sulfate, and the solvent was then removed by evaporation. The resultant intermediate was refluxed in diphenyl ether for 30 min. The reaction solution was allowed to stand for cooling, and the precipitate was then collected by filtration under the reduced pressure to give 4.0 g of a mixture of 2,3-dimethyl-4-hydroxy-6,7-difluoroquinoline with 2,3-dimethyl-4-hydroxy-5,6-difluoroquinoline. A 60% sodium hydride (230 mg) was suspended in 10 ml of tetrahydrofuran. Separately, the mixture of 2,3-dimethyl-4-hydroxy-6,7-difluoroquinoline with 2,3-dimethyl-4-hydroxy-5,6-difluoroquinoline (starting material 1) (1 g) was suspended in 20 ml of tetrahydrofuran, and this suspension of starting material 1 was added dropwise to the above suspension of sodium hydride under ice cooling. The reaction mixture was stirred for one hr. Cyclopropanecarbonyl chloride (starting material 2) (600 mg) was then added thereto, and the mixture was stirred at room temperature for 15 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate. The solvent was then removed by evaporation under the reduced pressure. The crude product thus obtained was purified on Wako Gel C-200 (200 ml; elution solvent = n-hexane-ethyl acetate (10 : 1)) to give 890 mg of 2,3-dimethyl-4-cyclopropanecarbonyloxy-6,7-difluoroquinoline (compound 15) and 90.6 mg of 2,3-dimethyl-4-cyclopropanecarbonyloxy-5,6-difluoroquinoline (compound 16). ¹H-NMR data were shown in Table 3.

### Alternative to Example 4

### Synthesis of starting material 1 (mixture of 2,3-dimethyl-4-hydroxy-6,7-difluoroquinoline with 2,3-dimethyl-4-hydroxy-5,6-difluoroquinoline)

3,4-Difluoroaniline (1.0 g) and 1.11 g of methyl-2-methyl acetoacetate were stirred in 5 ml of xylene in the presence of 0.3 ml of boron trifluoride etherate at 140°C for 3 hr. The reaction solution was allowed to stand for cooling, and the precipitate was then collected by filtration to give 906.8 mg of a mixture of 2,3-dimethyl-4-hydroxy-6,7-difluoroquinoline with 2,3-dimethyl-4-hydroxy-5,6-difluoroquinoline.

### Example 5: 2,3-Dimethyl-4-acetyloxy-6,7-difluoroquinoline (compound 17)

A 60% sodium hydride (230 mg) was suspended in 10 ml of tetrahydrofuran. Separately, the mixture (1 g) of 2,3-dimethyl-4-hydroxy-6,7-difluoroquinoline with 2,3-dimethyl-4-hydroxy-5,6-difluoroquinoline (starting material 1) prepared as described in Example 4 was suspended in 20 ml of tetrahydrofuran, and this suspension of starting material 1 was added dropwise to the above suspension of sodium hydride under ice cooling. The reaction mixture was stirred for one hr. Acetyl chloride (starting material 2) (450 mg) was added thereto, and the mixture was then stirred at room temperature for 15 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate. The solvent was then removed by evaporation under the reduced pressure. The crude product thus obtained was purified on Wako Gel C-200 (200 ml; elution solvent = n-hexane-ethyl acetate (10 : 1)) to give 262.5 mg of 2,3-dimethyl-4-acetyloxy-6,7-difluoroquinoline (compound 17). ¹H-NMR data were shown in Table 3.

### Example 6: 2-Ethyl-3-methyl-4-acetyloxy-6-fluoro-7-chloroquinoline (compound 18) and 2-ethyl-3-methyl-4-acetyloxy-5-chloro-6-fluoroquinoline (compound 19)

3-Chloro-4-fluoroaniline (2.91 g) and 3.16 g of ethyl-2-methylpropionyl acetate were refluxed in toluene (60 ml) in the presence of 0.3 ml of boron trifluoride etherate for 3 hr. The reaction mixture obtained was then washed with a saturated sodium hydrogencarbonate solution and saturated brine and was dried over anhydrous sodium sulfate, and the solvent was then removed by evaporation. The resultant intermediate was refluxed in diphenyl ether for 30 min. The reaction solution was allowed to stand for cooling, and the precipitate was then collected by filtration under the reduced pressure to give 820 mg of a mixture of 2-ethyl-3-methyl-4-hydroxy-6-fluoro-7-chloroquinoline with 2-ethyl-3-methyl-4-hydroxy-5-chloro-6-fluoroquinoline. A 60% sodium hydride (90.3 mg) was suspended in 10 ml of tetrahydrofuran. Separately, the mixture of 2-ethyl-3-methyl-4-hydroxy-6-fluoro-7-chloroquinoline with 2-ethyl-3-methyl-4-hydroxy-5-chloro-6-fluoroquinoline (starting material 1) (400 mg) was suspended in 10 ml of tetrahydrofuran, and this suspension was added dropwise to the above suspension of sodium hydride under ice cooling. The mixture was stirred for one hr. Acetyl chloride (starting material 2) (180 µl) was then added thereto, and the mixture was stirred at room temperature for 3 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate. The solvent was then removed by evaporation under the reduced pressure. The crude product thus obtained was purified on Wako Gel C-200 (100 ml; elution solvent = n-hexane-ethyl acetate (10 : 1)) to give 17.5 mg of 2-ethyl-3-methyl-4-acetyloxy-6-fluoro-7-chloro-quinoline (compound 18) and 44.6 mg of 2-ethyl-3-methyl-4-acetyloxy-5-chloro-6-fluoroquinoline (compound 19). ¹H-NMR data were shown in Table 3.

### Example 7: 2-Ethyl-3-methyl-4-cyclopropanecarbonyloxy-6-fluoro-7-chloroquinoline (compound 20) and 2-ethyl-3-methyl-4-cyclopropanecarbonyloxy-5-chloro-6-fluoroquinoline (compound 21)

A 60% sodium hydride (90.3 mg) was suspended in 5 ml of tetrahydrofuran. Separately, 400 mg of the mixture of 2-ethyl-3-methyl-4-hydroxy-6-fluoro-7-chloroquinoline with 2-ethyl-3-methyl-4-hydroxy-5-chloro-6-fluoroquinoline (starting material 1) prepared as described in Example 6 was suspended in 10 ml of tetrahydrofuran, and this suspension was added dropwise to the above suspension of sodium hydride under ice cooling. The mixture was stirred for one hr. Cyclopropanecarbonyl chloride (starting material 2) (240 mg) was then added thereto, and the mixture was stirred at room temperature for 15 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate, and the solvent was then removed by evaporation under the reduced pressure. The crude product thus obtained was purified on Wako Gel C-200 (200 ml; elution solvent = n-hexane-ethyl acetate (10 : 1)) to give 245.4 mg of 2-ethyl-3-methyl-4-cyclopropanecarbonyloxy-6-fluoro-7-chloroquinoline (compound 20) and 129.2 mg of 2-ethyl-3-methyl-4-cyclopropanecarbonyloxy-5-chloro-6-fluoroquinoline (compound 21). ¹H-NMR data were shown in Table 3.

### Example 8: 2-Ethyl-3-methyl-4-isopropoxycarbonyloxy-6-fluoro-7-chloroquinoline (compound 22) and 2-ethyl-3-methyl-4-isopropoxycarbonyloxy-5-chloro-6-fluoroquinoline (compound 23)

A 60% sodium hydride (96 mg) was suspended in 5 ml of tetrahydrofuran. Separately, the mixture of 2-ethyl-3-methyl-4-hydroxy-6-fluoro-7-chloroquinoline with 2-ethyl-3-methyl-4-hydroxy-5-chloro-6-fluoroquinoline (starting material 1) (480 mg) prepared as described in Example 6 was suspended in 10 ml of tetrahydrofuran, and this suspension was added dropwise to the above suspension of sodium hydride under ice cooling. After stirring for one hr, 300 mg of isopropyl chloroformate (starting material 2) was added thereto, and the mixture was stirred at room temperature for 15 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate. The solvent was then removed by evaporation under the reduced pressure. The crude product thus obtained was purified on Wako Gel C-200 (200 ml; elution solvent = n-hexane-ethyl acetate (10 : 1)) to give 345.1 mg of 2-ethyl-3-methyl-4-isopropoxycarbonyloxy-6-fluoro-7-chloroquinoline (compound 22) and 170.7 mg of 2-ethyl-3-methyl-4-isopropoxycarbonyloxy-5-chloro-6-fluoroquinoline (compound 23). ¹H-NMR data were shown in Table 3.

### Example 9: 2,3-Dimethyl-4-cyclopropanecarbonyloxy-6-fluoro-7-chloroquinoline (compound 26) and 2,3-dimethyl-4-cyclopropanecarbonyloxy-5-chloro-6-fluoroquinoline (compound 27)

3-Chloro-4-fluoroaniline (5.0 g) and 4.9 g of ethyl-2-methyl acetoacetate were refluxed in toluene (50 ml) in the presence of 0.3 ml of boron trifluoride etherate for 3 hr. The reaction mixture obtained was washed with a saturated sodium hydrogencarbonate solution and saturated brine and was dried over anhydrous sodium sulfate, and the solvent was then removed by evaporation. The resultant intermediate was refluxed in diphenyl ether for 30 min. The reaction mixture was allowed to stand for cooling, and the precipitate was then collected by filtration under the reduced pressure to give 1.7 g of a mixture of 2,3-dimethyl-4-hydroxy-6-fluoro-7-chloroquinoline with 2,3-dimethyl-4-hydroxy-5-chloro-6-fluoroquinoline. A 60% sodium hydride (350 mg) was suspended in 50 ml of dimethyl formamide. Separately, the mixture of 2,3-dimethyl-4-hydroxy-6-fluoro-7-chloroquinoline with 2,3-dimethyl-4-hydroxy-5-chloro-6-fluoroquinoline (starting material 1) (1.7 g) was suspended in 30 ml of dimethyl formamide, and this suspension was added dropwise to the above suspension of sodium hydride under ice cooling. After stirring for one hr, 940 µl of cyclopropanecarbonyl chloride (starting material 2) was added thereto, and the mixture was stirred at room temperature for 13 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate. The solvent was then removed by evaporation under the reduced pressure. The crude product thus obtained was purified on Wako Gel C-200 (100 ml; elution solvent = n-hexane-ethyl acetate (10 : 1)) to give 480 mg of 2,3-dimethyl-4-cyclopropanecarbonyloxy-6-fluoro-7-chloroquinoline (compound 26) and 350 mg of 2,3-dimethyl-4-cyclopropanecarbonyloxy-5-chloro-6-fluoro-quinoline (compound 27). ¹H-NMR data were shown in Table 3.

### Example 10: 2,3-Dimethyl-4-methoxycarbonyloxy-6-fluoro-7-chloroquinoline (compound 28)

2,3-Dimethyl-4-cyclopropanecarbonyloxy-6-fluoro-7-chloroquinoline (590 mg) prepared as described in Example 9 was dissolved in 5 ml of methanol, and 5 ml of a 10% aqueous sodium hydroxide solution was added to this solution. The mixture obtained was stirred at 50°C for 3 hr. The reaction mixture was allowed to stand for cooling, was then poured into 50 ml of water, and was neutralized with 1 N hydrochloric acid, and the precipitate was then collected by filtration to give 400 mg of 2,3-dimethyl-4-hydroxy-6-fluoro-7-chloroquinoline. A 60% sodium hydride (26.4 mg) was suspended in 3 ml of tetrahydrofuran. Separately, 100 mg of 2,3-dimethyl-4-hydroxy-6-fluoro-7-chloroquinoline (starting material 1) prepared above was suspended in 2 ml of tetrahydrofuran, and this suspension was added dropwise to the above suspension of sodium hydride under ice cooling. After stirring for one hr, 0.1 ml of methyl chloroformate (starting material 2) was added thereto, and the mixture was stirred at room temperature for 3 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate. The solvent was then removed by evaporation under the reduced pressure. The crude product thus obtained was purified on Wako Gel C-200 (100 ml; elution solvent = n-hexane-ethyl acetate (10 : 1)) to give 48 mg of 2,3-dimethyl-4-methoxycarbonyloxy-6-fluoro-7-chloroquinoline (compound 28). ¹H-NMR data were shown in Table 3.

### Example 11: 2-Ethyl-3-methyl-4-methoxycarbonyloxy-5-chloro-6-fluoroquinoline (compound 36)

2-Ethyl-3-methyl-4-cyclopropanecarbonyloxy-5-chloro-6-fluoroquinoline (1.5 g) prepared as described in Example 7 was dissolved in 70 ml of methanol. To this solution was added 30 ml of a 10% aqueous sodium hydroxide solution. The mixture obtained was stirred at 50°C for 1.5 hr. The reaction mixture was allowed to stand for cooling, was then poured into 50 ml of water, and was neutralized with 1 N hydrochloric acid, and the precipitate was then collected by filtration to give 1.17 g of 2-ethyl-3-methyl-4-hydroxy-5-chloro-6-fluoroquinoline. A 60% sodium hydride (40.1 mg) was suspended in 15 ml of dimethyl formamide. Separately, 200 mg of 2-ethyl-3-methyl-4-hydroxy-5-chloro-6-fluoroquinoline (starting material 1) prepared above was suspended in 2 ml of dimethyl formamide, and this suspension was added dropwise to the above suspension of sodium hydride under ice cooling. After stirring for one hr, 78.5 mg of methyl chloroformate (starting material 2) was added thereto, and the mixture was stirred at room temperature for 3 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate. The solvent was then removed by evaporation under the reduced pressure. The crude product thus obtained was purified on Wako Gel C-200 (100 ml; elution solvent = n-hexane-ethyl acetate (10 : 1)) to give 218.8 mg of 2-ethyl-3-methyl-4-methoxycarbonyloxy-5-chloro-6-fluoroquinoline (compound 36). ¹H-NMR data were shown in Table 3.

### Example 12: 2,3-Dimethyl-4-acetoxy-5,6-difluoroquinoline (compound 44)

2,3-Dimethyl-4-cyclopropanecarbonyloxy-5,6-difluoroquinoline (1.5 g) prepared as described in Example 4 was dissolved in 15 ml of methanol. A 10% aqueous sodium hydroxide solution (15 ml) was added to the solution, and the mixture was stirred at 50°C for 1.5 hr. The reaction mixture obtained was allowed to stand for cooling, was then poured into 50 ml of water, and was neutralized with 1 N hydrochloric acid, and the precipitate was then collected by filtration to give 1.1 g of 2,3-dimethyl-4-hydroxy-5,6-difluoroquinoline. A 60% sodium hydride (11.5 mg) was suspended in 5 ml of dimethyl formamide. Separately, 50 mg of 2,3-dimethyl-4-hydroxy-5,6-difluoroquinoline (starting material 1) prepared above was suspended in 5 ml of dimethyl formamide, and this suspension was added dropwise to the above suspension of sodium hydride under ice cooling. After stirring for one hr, 20 mg of acetyl chloride (starting material 2) was added thereto, and the mixture was stirred at room temperature for 15 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate. The solvent was then removed by evaporation under the reduced pressure. The crude product thus obtained was purified on Wako Gel C-200 (100 ml; elution solvent = n-hexane-ethyl acetate (10 : 1)) to give 54.2 mg of 2,3-dimethyl-4-acetoxy-5,6-difluoroquinoline (compound 44). ¹H-NMR data were shown in Table 3.

### Example 13: 2,3-Dimethyl-4-(3-transchloroacryloyloxy)-5,6-difluoroquinoline (compound 53) and 2,3-dimethyl-4-(3-cischloroacryloyloxy)-5,6-difluoroquinoline (compound 54)

Thionyl chloride (300 µl) was added to 213 mg of 3-cischloroacrylic acid, and the mixture was stirred at 60°C for one hr. Thionyl chloride was removed by evaporation under the reduced pressure to give 3-chloroacrylic acid chloride.

A 60% sodium hydride (11.5 mg) was suspended in 5 ml of dimethyl formamide. Separately, 50 mg of 2,3-dimethyl-4-hydroxy-5,6-difluoroquinoline (starting material 1) prepared as described in Example 12 was suspended in 5 ml of dimethyl formamide, and this suspension was added dropwise to the above suspension of sodium hydride under ice cooling. After stirring for one hr, 50 mg of 3-chloroacrylic acid chloride (starting material 2) was added thereto, and the mixture was stirred at room temperature for 15 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate. The solvent was then removed by evaporation under the reduced pressure. The crude product thus obtained was purified on Wako Gel C-200 (100 ml; elution solvent = n-hexane-ethyl acetate (10 : 1)) to give 13.6 mg of 2,3-dimethyl-4-(3-transchloroacryloyloxy)-5,6-difluoroquinoline (compound 53) and 10.3 mg of 2,3-dimethyl-4-(3-cischloroacryloyloxy)-5,6-difluoro-quinoline (compound 54). ¹H-NMR data were shown in Table 3.

### Example 14: 2,3-Dimethyl-4-cyclopropanecarbonyloxy-5-trifluoromethyl-6-fluoroquinoline (compound 61) and 2,3-dimethyl-4-cyclopropanecarbonyloxy-6-fluoro-7-trifluoromethylquinoline (compound 63)

4-Fluoro-3-trifluoromethylaniline (1.0 g) and 0.81 g of methyl-2-methyl acetoacetate were stirred in xylene in the presence of 0.3 ml of boron trifluoride etherate at 140°C for 3 hr. The reaction solution obtained was allowed to stand for cooling, and the precipitate was then collected by filtration to give 228.2 mg of a mixture of 2,3-dimethyl-4-hydroxy-5-trifluoromethyl-6-fluoro-quinoline with 2,3-dimethyl-4-hydroxy-6-fluoro-7-trifluoromethylquinoline.

A 60% sodium hydride (18.8 mg) was suspended in 3 ml dimethyl formamide. Separately, the mixture of 2,3-dimethyl-4-hydroxy-5-trifluoromethyl-6-fluoroquinoline with 2,3-dimethyl-4-hydroxy-6-fluoro-7-trifluoromethylquinoline (starting material 1) (100 mg) was suspended in 3 ml of dimethyl formamide, and this suspension was added dropwise to the above suspension of sodium hydride under ice cooling. After stirring for one hr, 40.8 mg of cyclopropanecarbonyl chloride (starting material 2) was added thereto, and the mixture was stirred at room temperature for 15 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate. The solvent was then removed by evaporation under the reduced pressure. The crude product thus obtained was purified on a silica gel packed column (Varian) (20 g; elution solvent = n-hexane-ethyl acetate (40 : 1) to (20 : 1)) to give 27.9 mg of 2,3-dimethyl-4-cyclopropanecarbonyloxy-5-trifluoromethyl-6-fluoroquinoline (compound 61) and 51.3 mg of 2,3-dimethyl-4-cyclopropanecarbonyloxy-6-fluoro-7-trifluoro-methylquinoline (compound 63). ¹H-NMR data were shown in Table 3.

### Example 15: 2,3-Dimethyl-4-cyclopropanecarbonyloxy-5,6-dichloroquinoline (compound 65) and 2,3-dimethyl-4-cyclopropanecarbonyloxy-6,7-dichloroquinoline (compound 68)

3,4-Dichloroaniline (1.0 g) and 0.89 g of methyl-2-methyl acetoacetate were stirred in xylene in the presence of 0.3 ml of boron trifluoride etherate at 140°C for 3 hr. The reaction solution obtained was allowed to stand for cooling, and the precipitate was then collected by filtration to give 667.4 mg of a mixture of 2,3-dimethyl-4-hydroxy-5,6-dichloroquinoline with 2,3-dimethyl-4-hydroxy-6,7-dichloroquinoline.

A 60% sodium hydride (40.0 mg) was suspended in 3 ml of dimethyl formamide. Separately, the mixture of 2,3-dimethyl-4-hydroxy-5,6-dichloroquinoline with 2,3-dimethyl-4-hydroxy-6,7-dichloroquinoline (starting material 1) (200 mg) was suspended in 3 ml of dimethyl formamide, and this suspension was added dropwise to the above suspension of sodium hydride under ice cooling. After stirring for one hr, 86.7 mg of cyclopropanecarbonyl chloride (starting material 2) was added thereto, and the mixture was stirred at room temperature for 15 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate. The solvent was then removed by evaporation under the reduced pressure. The crude product thus obtained was purified on Wako Gel C-200 (100 ml; elution solvent = n-hexane-ethyl acetate (10 : 1)) to give 75.2 mg of 2,3-dimethyl-4-cyclopropanecarbonyloxy-5,6-dichloroquinoline (compound 65) with 4 mg of 2,3-dimethyl-4-cyclopropanecarbonyloxy-6,7-dichloroquinoline (compound 68). ¹H-NMR data were shown in Table 3.

### Example 16: 2,3-Dimethyl-4-(5-hexynoylcarbonyloxy)-5,6-difluoroquinoline (compound 58)

5-Hexynoic acid (starting material 2) (26.9 mg), 40.6 mg of 2-chloro-1,3-dimethylimidazolinium chloride and 37.9 mg of pyridine were stirred in dichloromethane (5 ml) at room temperature for one hr. Thereafter, 50 mg of 2,3-dimethyl-4-hydroxy-5,6-difluoroquinoline (starting material 1) prepared as described in Example 12 was added thereto, and the mixture was stirred at room temperature for 15 hr. Water was added to the reaction solution, and the mixture was extracted with dichloromethane. The dichloromethane layer obtained was washed with a saturated aqueous sodium hydrogencarbonate solution and was then dried over anhydrous sodium sulfate. The solvent was then removed by evaporation under the reduced pressure. The crude product thus obtained was purified on Wako Gel C-200 (10 ml; elution solvent = n-hexane-ethyl acetate (10 : 1)) to give 77.8 mg of 2,3-dimethyl-4-(5-hexynoylcarbonyloxy)-5,6-difluoroquinoline (compound 58). ¹H-NMR data were shown in Table 3.

### Example 17: 2-Ethyl-3-methyl-4-methanethiolcarbonyloxy-5,6-difluoroquinoline (compound 39)

2-Ethyl-3-methyl-4-hydroxy-5,6-difluoroquinoline (starting material 1) (30 mg) prepared as described in Example 3 was suspended in dichloromethane under an argon atmosphere in the presence of dimethylaminopyridine. Triethylamine (16 mg) was added thereto, and the mixture was stirred at room temperature for 30 min. Thereafter, 16 mg of methyl chlorothioformate (starting material 2) was added thereto, and the mixture was stirred overnight. The reaction solution as such was concentrated, and the residue was then purified on Wako Gel C-200 (10 ml; elution solvent = n-hexane-ethyl acetate (10 : 1)) to give 40 mg of 2-ethyl-3-methyl-4-methanethiolcarbonyloxy-5,6-difluoroquinoline (compound 39). ¹H-NMR data were shown in Table 3.

### Example 18: 2-Isopropyl-3-methyl-4-cyclopropanecarbonyloxy-6,7-difluoroquinoline (compound 34)

3,4-Difluoroaniline (4.5 g) and 6 g of ethyl-2-methyl isovalerylacetate were refluxed in toluene (50 ml) in the presence of 0.3 ml of boron trifluoride etherate for 3 hr. The reaction mixture obtained was washed with a saturated sodium hydrogencarbonate solution and saturated brine and was dried over anhydrous sodium sulfate, and the solvent was then removed by evaporation. The resultant intermediate was refluxed in diphenyl ether for 30 min. The reaction mixture was allowed to stand for cooling, and the precipitate was then collected by filtration under the reduced pressure to give 246 mg of a mixture of 2-isopropyl-3-methyl-4-hydroxy-6,7-difluoroquinoline with 2-isopropyl-3-methyl-4-hydroxy-5,6-difluoroquinoline. A 60% sodium hydride (20 mg) was suspended in 10 ml of dimethyl formamide. Separately, 100 mg of the mixture of 2-isopropyl-3-methyl-4-hydroxy-6,7-difluoroquinoline with 2-isopropyl-3-methyl-4-hydroxy-5,6-difluoroquinoline (starting material 1) was suspended in 20 ml of dimethyl formamide, and this suspension was added dropwise to the above suspension of sodium hydride under ice cooling. After stirring for one hr, 100 mg of cyclopropanecarbonyl chloride (starting material 2) was added thereto, and the mixture was stirred at room temperature for 3 hr. The reaction solution was poured into water, and the mixture was extracted with ethyl acetate. The ethyl acetate layer obtained was washed with a saturated aqueous sodium hydrogencarbonate solution and saturated brine and was then dried over anhydrous sodium sulfate. The solvent was then removed by evaporation under the reduced pressure. The crude product thus obtained was purified on Wako Gel C-200 (100 ml; elution solvent = n-hexane-ethyl acetate (10 : 1)) to give 44.1 mg of 2-isopropyl-3-methyl-4-cyclopropanecarbonyloxy-6,7-difluoroquinoline (compound 34). ¹H-NMR data were shown in Table 3.

Chemical structures in formula (I) of compounds 1 to 3, 12, 15 to 23, 26 to 28, 34, 36, 39, 44, 53, 54, 58, 61, 63, 65, and 68 synthesized in Examples 1 to 18 were as follows.

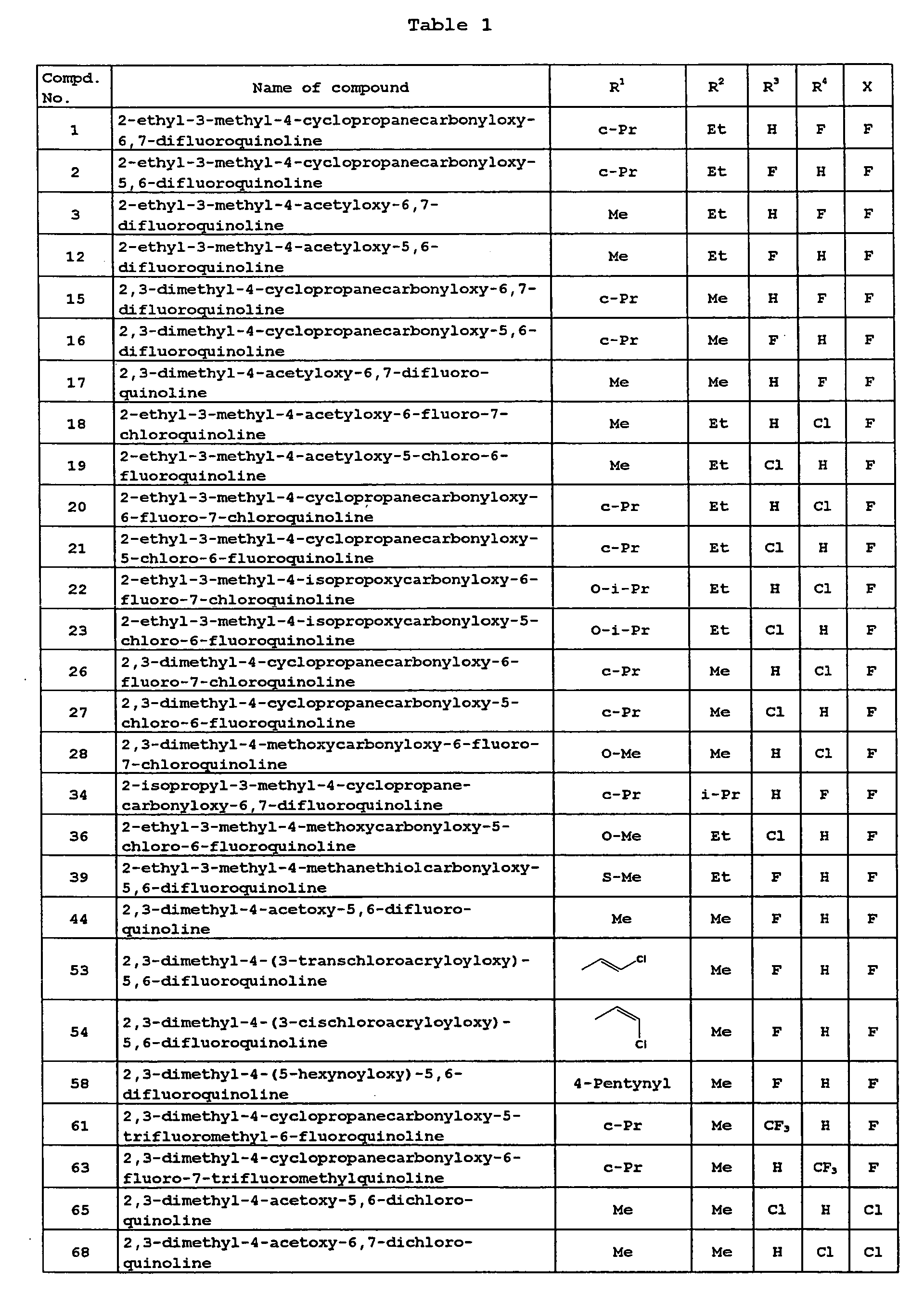

Compounds 4 to 11, 13, 14, 24, 25, 29 to 33, 35, 37, 38, 40 to 43, 45 to 52, 55 to 57, 59, 60, 62, 64, 66, 67, and 69 to 81 were synthesized as described in the above examples. The name of compounds, starting materials, yields, and chemical structures in formula (I) were as follows.

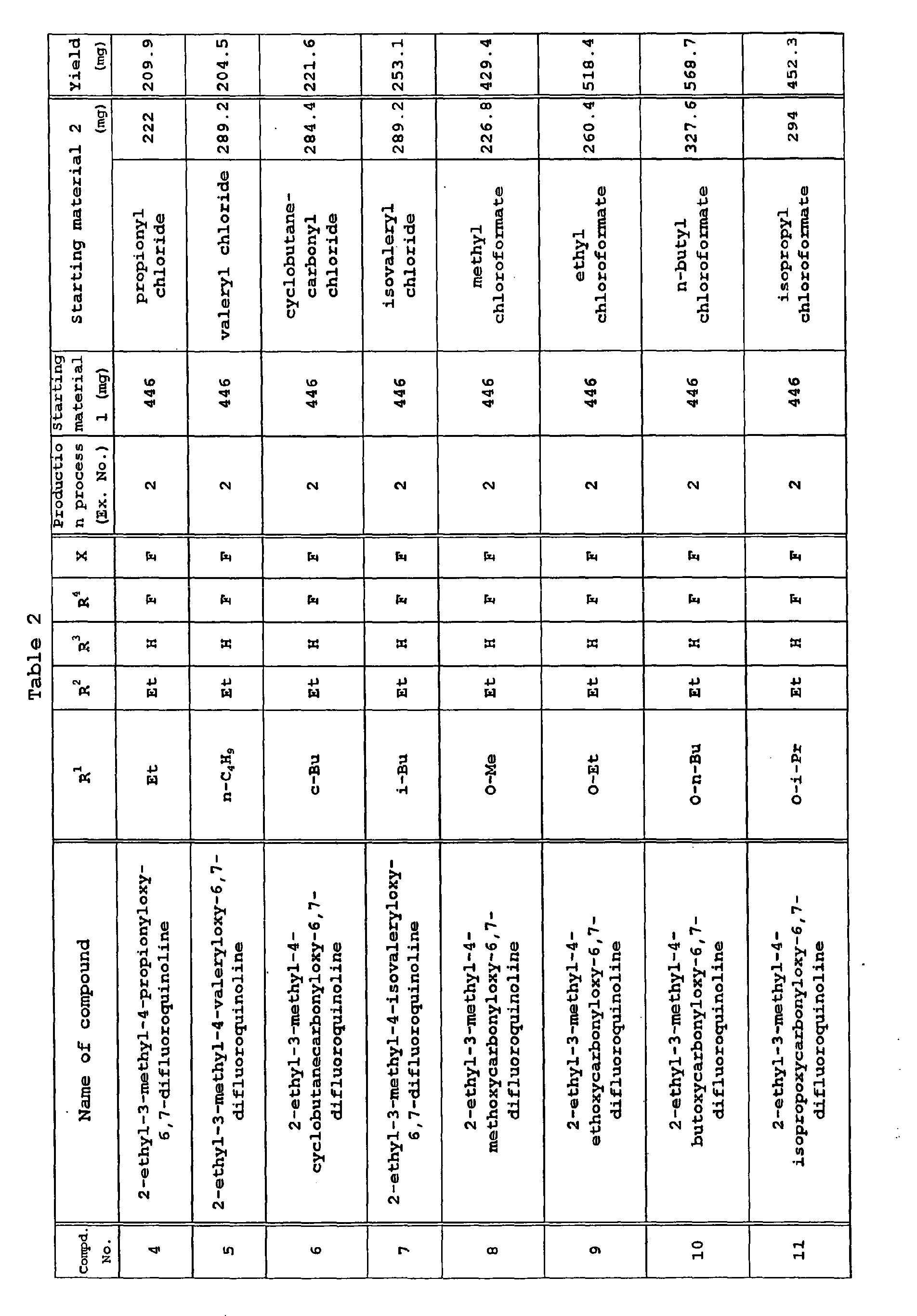

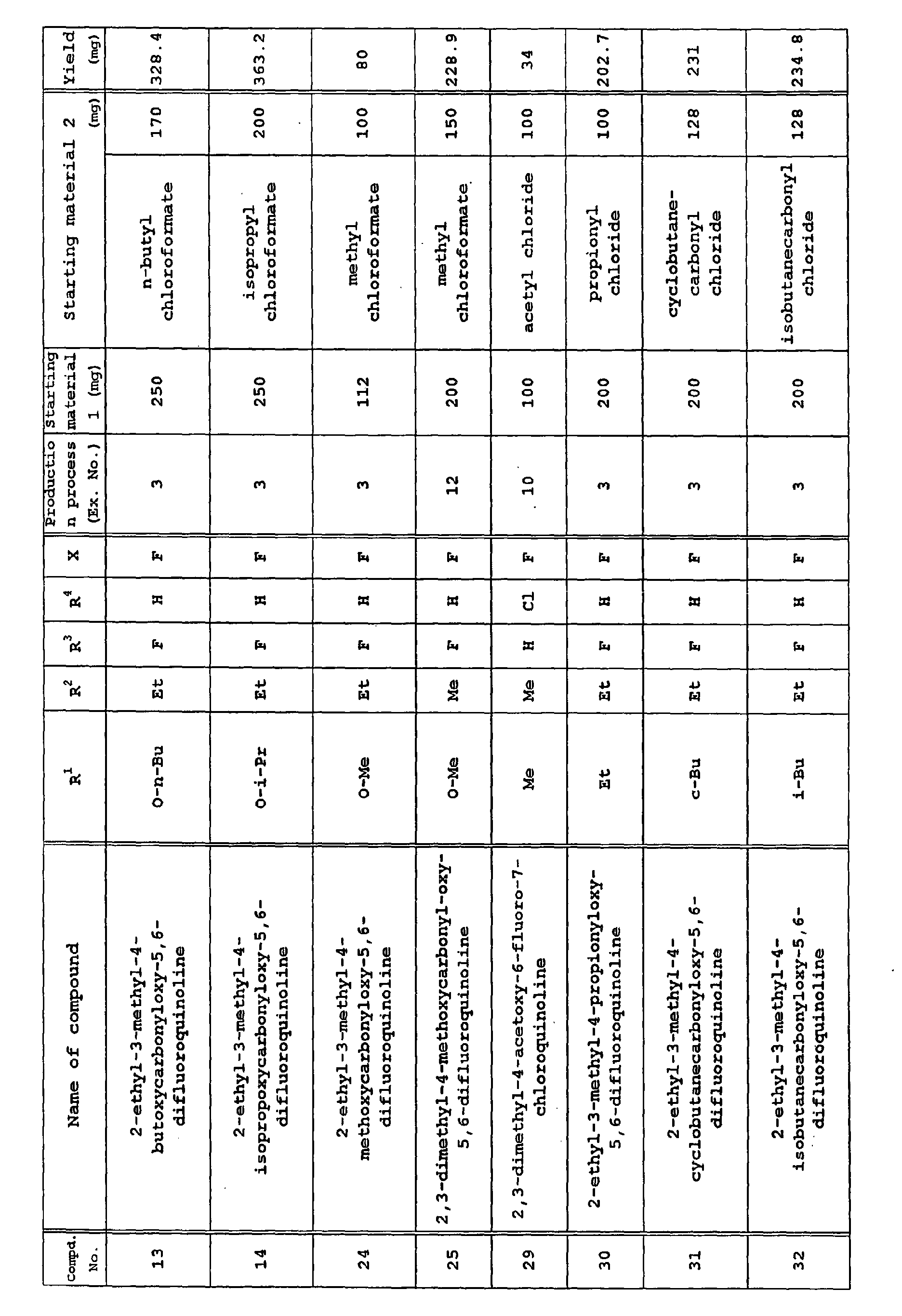

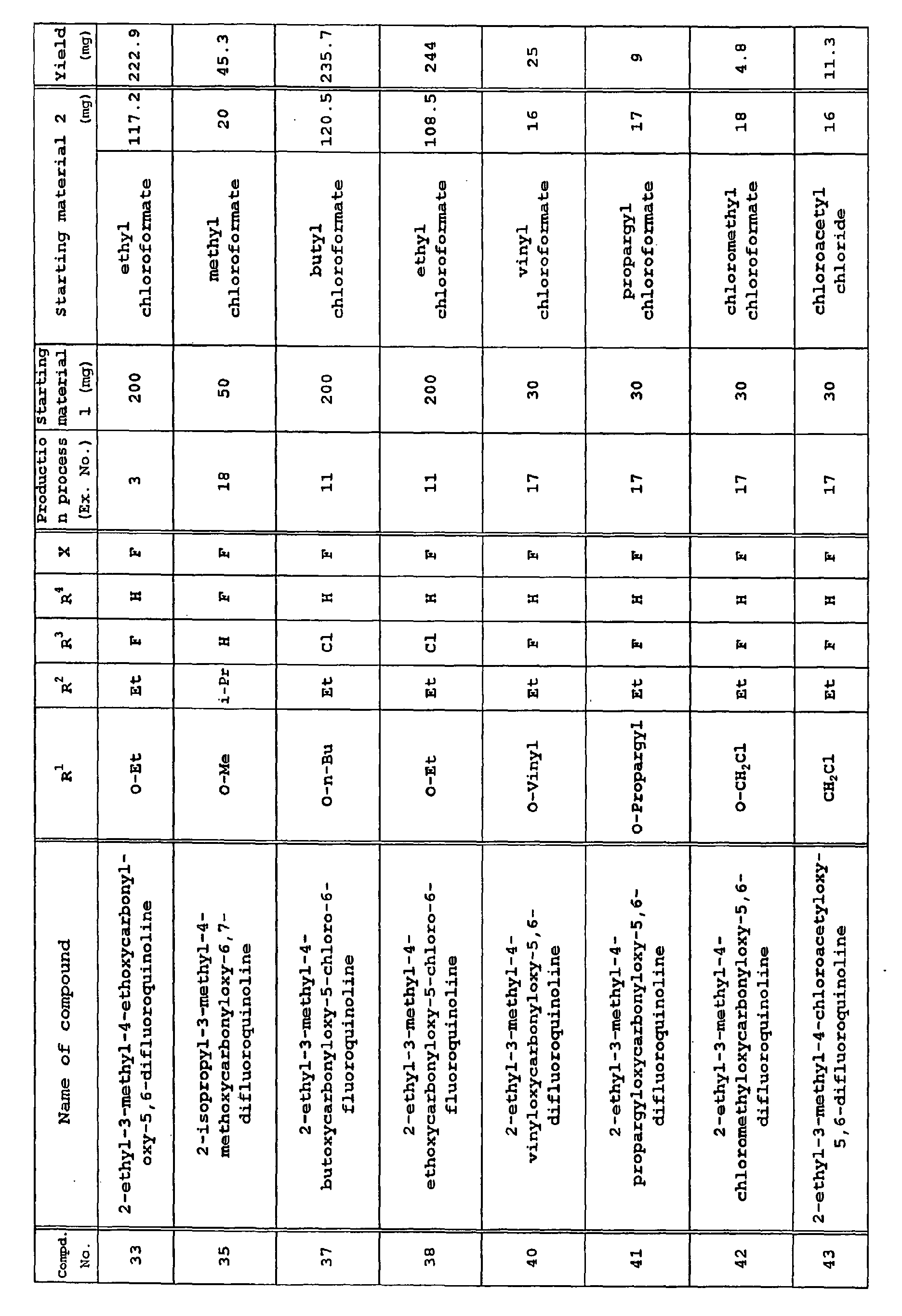

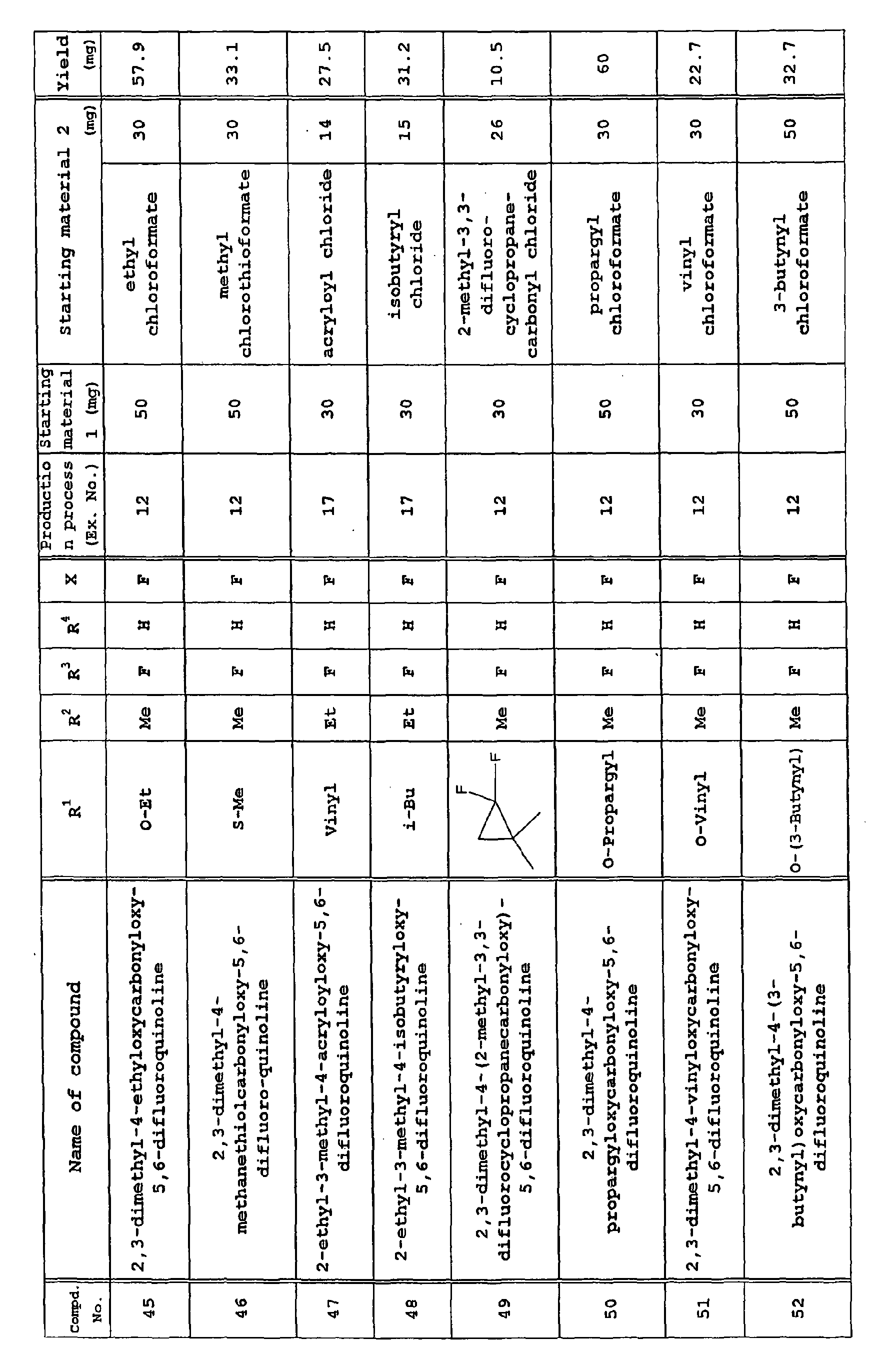

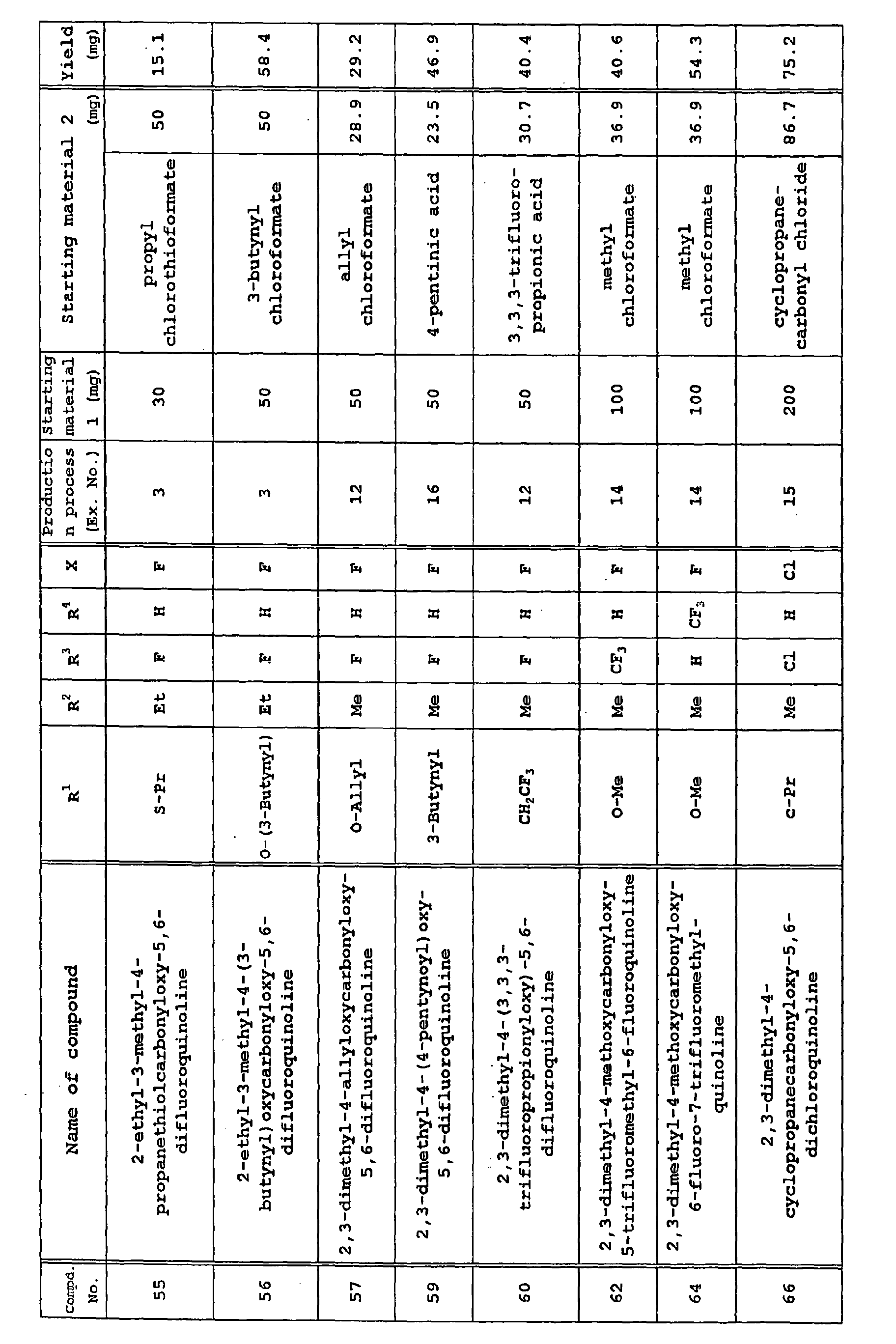

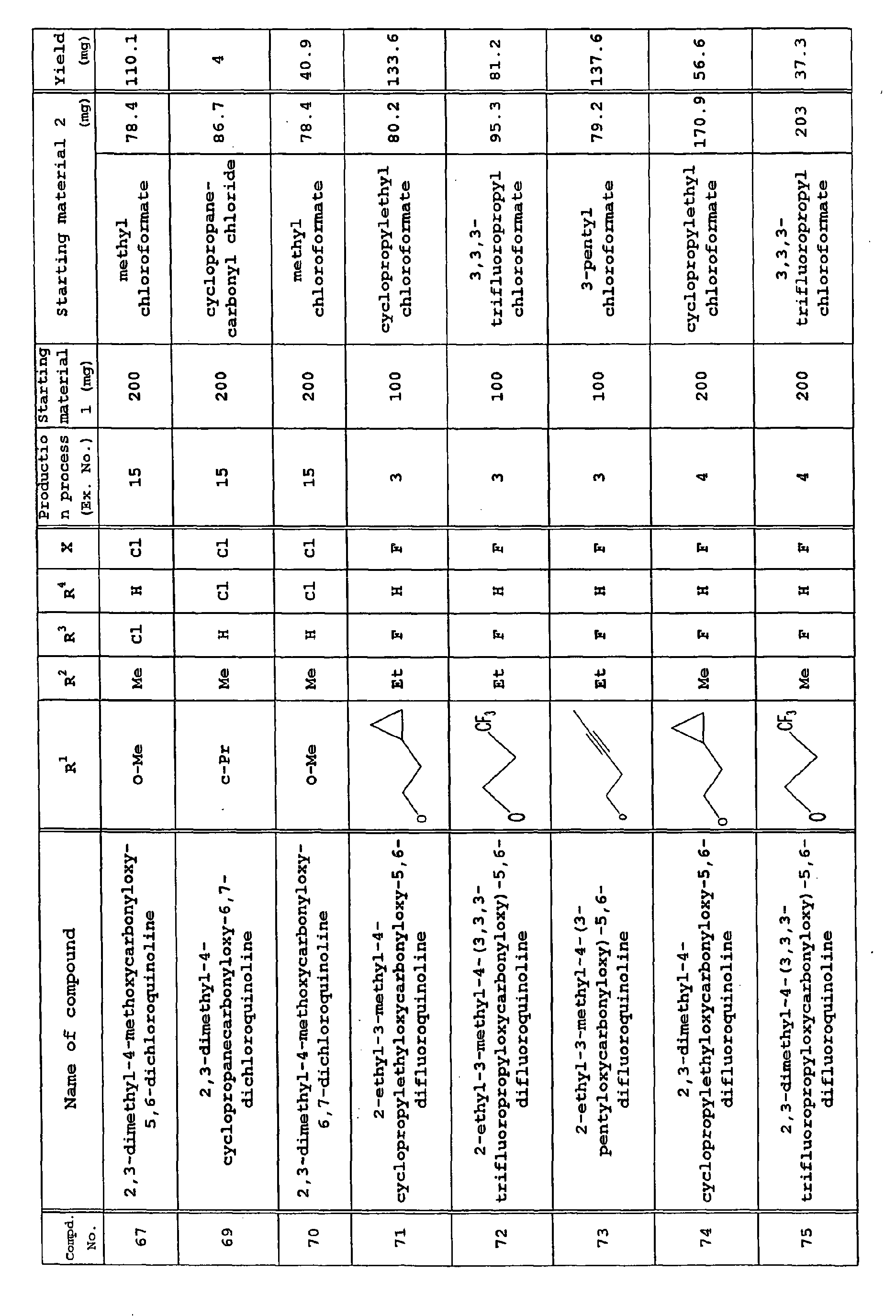

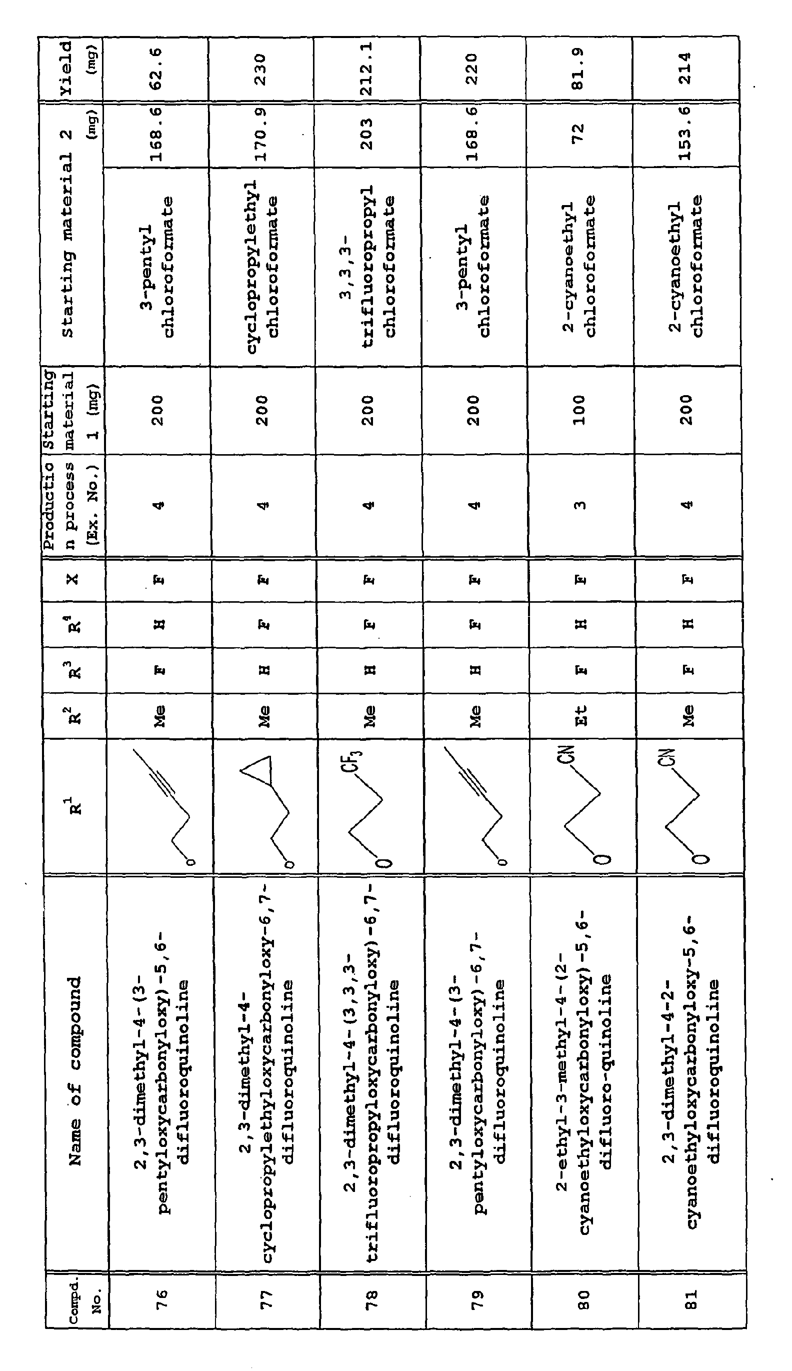

¹H-NMR data of compounds 1 to 81 were as follows.

**Table 3**

| Compound No. | ¹H-NMR data; δ (ppm) from TMS in CDCL₃ |
|---|---|
| 1 | 7.78 (1H, dd, J1 = 11.2 Hz, J2 = 7.6 Hz), 7.43 (1H, dd, J1 = 10.5 Hz, J2 = 8.3 Hz), 2.99 (2H, q, J = 7.6 Hz), 2.26 (3H, s), 2.06 (1H, m), 1.36 (3H, t, J = 7.5 Hz), 1.29 (2H, m), 1.18 (2H, m) |
| 2 | 7.79 (1H, m), 7.45 (1H, dd, J1 = 17.8 Hz, J2 = 9.7 Hz), 2.99 (2H, q, J = 7.6 Hz), 2.30 (3H, s), 2.01 (1H, m), 1.37 (3H, t, J = 7.6 Hz), 1.29 (2H, m), 1.13 (2H, m) |
| 3 | 7.80 (1H, m), 7.40 (1H, dd, J1 = 10.5 Hz, J2 = 8.2 Hz), 3.00 (2H, q, J = 7.5 Hz), 2.51 (3H, s), 2.67 (3H, s), 1.37 (3H, t, J = 7.5 Hz) |
| 4 | 7.78 (1H, m), 7.37 (1H, dd, J1 = 10.7 Hz, J2 = 8.3 Hz), 2.99 (2H, q, J = 7.6 Hz), 2.81 (2H, q, J = 7.5 Hz), 2.25 (3H, s), 1.40 (3H, t, J = 7.5 Hz), 1.37 (3H, t, J = 7.5 Hz) |
| 5 | 7.79 (1H, m), 7.38 (1H, dd, J1 = 10.7 Hz, J2 = 8.5 Hz), 2.99 (2H, q, J = 7.5 Hz), 2.77 (2H, t, J = 7.3 Hz), 2.25 (3H, s), 1.86 (2H, m), 1.52 (2H, m), 1.37 (3H, t, J = 7.5 Hz), 1.03 (3H, t, J = 7.3 Hz) |
| 6 | 7.80 (1H, m), 7.37 (1H, dd, J1 = 10.7 Hz, J2 = 8.6 Hz), 3.62 (1H, m), 3.00 (2H, m), 2.56 (2H, m), 2.48 (2H, m), 2.25 (3H, s), 2.15 (2H, m), 1.37 (3H, t, J = 7.6 Hz) |
| 7 | 7.79 (1H, m), 7.37 (1H, dd, J1 = 10.7 Hz, J2 = 8.2 Hz), 2.99 (2H, q, J = 7.5 Hz), 2.66 (2H, d, J = 7.1 Hz), 2.34 (1H, m), 2.26 (3H, s), 1.37 (3H, t, J = 7.6 Hz), 1.14 (6H, d, J = 6.6 Hz) |
| 8 | 7.80 (1H, dd, J1 = 11.2 Hz, J2 = 7.6 Hz), 7.50 (1H, dd, J1 = 10.5 Hz, J2 = 8.3 Hz), 4.00 (3H, s), 3.00 (2H, q, J = 7.5 Hz), 2.32 (3H, s), 1.37 (3H, t, J = 7.6 Hz) |
| 9 | 7.78 (1H, dd, J1 = 11.2 Hz, J2 = 7.5 Hz), 7.51 (1H, dd, J1 = 10.5 Hz, J2 = 8.3 Hz), 4.39 (2H, q, J = 7.0 Hz), 2.99 (2H, q, J = 7.5 Hz), 2.32 (3H, s), 1.45 (3H, t, J = 7.0 Hz), 1.37 (3H, t, J = 7.4 Hz) |
| 10 | 7.79 (1H, dd, J1 = 10.9 Hz, J2 = 7.5 Hz), 7.50 (1H, dd, J1 = 10.7 Hz, J2 = 8.3 Hz), 4.33 (2H, t, J = 6.8 Hz), 2.99 (2H, q, J = 7.5 Hz), 2.31 (3H, s), 1.78 (2H, m), 1.48 (2H, m), 1.37 (3H, t, J = 7.5 Hz), 1.00 (3H, t, J = 7.5 Hz) |
| 11 | 7.78 (1H, dd, J1 = 11.2 Hz, J2 = 7.6 Hz), 7.50 (1H, dd, J1 = 10.7 Hz, J2 = 8.5 Hz), 5.02 (1H, m), 2.99 (2H, q, J = 7.5 Hz), 2.31 (3H, s), 1.43 (6H, d, J = 6.4 Hz), 1.37 (3H, t, J = 7.5 Hz) |
| 12 | 7.81 (1H, m), 7.46 (1H, m), 2.99 (2H, q, J = 7.3 Hz), 2.45 (3H, s), 2.30 (3H, s), 1.37 (3H, t, J = 7.6 Hz) |
| 13 | 7.82 (1H, m), 7.47 (1H, dd, J1 = 17.8 Hz, J2 = 9.5 Hz), 4.33 (2H, t, J = 6.6 Hz), 3.00 (2H, q, J = 7.6 Hz), 2.36 (3H, s), 1.78 (2H, m), 1.49 (2H, m), 1.38 (3H, t, J = 7.5 Hz), 0.99 (3H, t, J = 7.3 Hz) |
| 14 | 7.83 (1H, m), 7.48 (1H, dd, J1 = 18.0 Hz, J2 = 9.7 Hz), 5.03 (1H, m), 3.00 (2H, q, J = 7.5 Hz), 1.43 (6H, d, J = 6.3 Hz), 0.73 (3H, t, J = 7.5 Hz) |
| 15 | 7.74 (1H, dd, J1 = 11.0 Hz, J2 = 7.7 Hz), 7.43 (1H, dd, J1 = 10.8 Hz, J2 = 8.6 Hz), 2.69 (3H, s), 2.23 (3H, s), 2.05 (1H, m), 1.29 (2H, m), 1.18 (2H, m) |
| 16 | 7.77 (1H, m), 7.45 (1H, m), 2.70 (3H, s), 2.28 (3H, s), 2.01 (1H, m), 1.28 (2H, m), 1.13 (2H, m) |
| 17 | 7.75 (1H, dd, J1 = 11.2 Hz, J2 = 7.5 Hz), 7.40 (1H, dd, J1 = 10.7 Hz, J2 = 8.2 Hz), 2.70 (3H, s), 2.51 (3H, s), 2.24 (3H, s) |
| 18 | 8.13 (1H, d, J = 7.1 Hz), 7.37 (1H, d, J = 9.3 Hz), 2.99 (2H, q, J = 7.4 Hz), 2.51 (3H, s), 2.27 (3H, s), 1.37 (3H, t, J = 7.6 Hz) |
| 19 | 7.98 (1H, dd, J1 = 9.3 Hz, J2 = 5.4 Hz), 7.47 (1H, t, J = 9 Hz), 3.00 (2H, q, J = 7.5 Hz), 2.48 (3H, s), 2.28 (3H, s), 1.37 (3H, t, J = 7.6 Hz) |
| 20 | 8.11 (1H, d, J = 7.1 Hz), 7.59 (1H, d, J = 9.3 Hz), 2.98 (2H, q, J = 7.6 Hz), 2.26 (3H, s), 2.04 (1H, m), 1.36 (3H, t, J = 7.6 Hz), 1.29 (2H, m), 1.18 (2H, m) |
| 21 | 8.00 (1H, m), 7.46 (1H, t, J = 9.0 Hz), 2.99 (2H, q, J = 7.5 Hz), 2.29 (3H, s), 2.06 (1H, m), 1.37 (3H, t, J = 7.6 Hz), 1.27 (2H, m), 1.14 (2H, m) |
| 22 | 8.12 (1H, d, J = 7.1 Hz), 7.69 (1H, d, J = 9.2 Hz), 5.02 (1H, m), 2.99 (2H, q, J = 7.3 Hz), 2.31 (3H, s), 1.43 (6H, d, J = 6.1 Hz), 1.37 (3H, t, J = 7.5 Hz) |
| 23 | 7.98 (1H, m), 7.46 (1H, t, J = 8.9 Hz), 5.04 (1H, m), 3.00 (2H, q, J = 7.4 Hz), 2.34 (3H, s), 1.42 (6H, d, J = 6.4 Hz), 1.37 (3H, t, J = 7.5 Hz) |
| 24 | 7.83 (1H, m), 7.49 (1H, dd, J1 = 13.9 Hz, J2 = 9.8 Hz), 3.99 (3H, s), 3.00 (2H, t, J = 7.3 Hz), 2.37 (3H, s), 1.38 (3H, t, J = 7.5 Hz) |
| 25 | 7.81 (1H, m), 7.49 (1H, dd, J1 = 17.7 Hz, J2 = 9.5 Hz), 3.99 (3H, s), 2.71 (3H, s), 2.34 (3H, s) |
| 26 | 8.09 (1H, d, J = 7.0 Hz), 7.42 (1H, d, J = 9.3 Hz), 2.70 (3H, s), 2.24 (3H, s), 2.05 (1H, m), 1.29 (2H, m), 1.18 (2H, m) |
| 27 | 7.90 (1H, m), 7.47 (1H, t, J = 8.8 Hz), 2.72 (3H, s), 2.27 (3H, s), 2.07 (1H, m), 1.28 (2H, m), 1.15 (2H, m) |
| 28 | 8.14 (1H, brs), 7.50 (1H, d, J = 9.3 Hz), 4.00 (3H, s), 2.72 (3H, s), 2.31 (3H, s) |
| 29 | 8.09 (1H, d, J = 7.0 Hz), 7.38 (1H, d, J = 9.5 Hz), 2.70 (3H, s), 2.51 (3H, s), 2.24 (3H, s) |
| 30 | 7.81 (1H, m), 7.47 (1H, dd, J1 = 18.1 Hz, J2 = 9.8 Hz), 3.00 (2H, q, J = 7.5 Hz), 2.77 (2H, q, J = 7.5 Hz), 2.29 (3H, s), 1.37 (3H, t, J = 7.5 Hz), 1.35 (3H, t, J = 7.5 Hz) |
| 31 | 7.82 (1H, m), 7.46 (1H, dd, J1 = 18.2 Hz, J2 = 9.3 Hz), 3.56 (1H, m), 3.00 (2H, m), 2.57 (2H, m), 2.42 (2H, m), 2.28 (3H, s), 2.10 (2H, m), 1.37 (3H, t, J = 7.6 Hz) |
| 32 | 7.81 (1H, m), 7.45 (1H, dd, J1 = 18.1 Hz, J2 = 9.6 Hz), 2.99 (2H, q, J = 7.3 Hz), 2.63 (2H, d, J = 7.0 Hz), 2.32 (1H, m), 2.30 (3H, s), 1.37 (3H, t, J = 7.6 Hz), 1.11 (6H, d, J = 6.8 Hz) |
| 33 | 7.83 (1H, m), 7.49 (1H, dd, J1 = 18.0 Hz, J2 = 9.7 Hz), 4.40 (2H, q, J = 7.1), 3.01 (2H, q, J = 7.6 Hz), 2.37 (3H, s), 1.44 (3H, t, J = 7.2 Hz), 1.38 (3H, t, J = 7.6 Hz) |
| 34 | 7.79 (1H, m), 7.40 (1H, dd, J1 = 10.7 Hz, J2 = 8.4 Hz), 3.40 (1H, m), 2.28 (3H, s), 2.03 (1H, m), 1.35 (6H, d, J = 6.6 Hz), 1.18 (2H, m), 1.13 (2H, m) |
| 35 | 7.83 (1H, m), 7.48 (1H, dd, J1 = 10.7 Hz, J2 = 8.5 Hz), 4.00 (3H, s), 3.41 (1H, m), 2.34 (3H, s), 1.36 (6H, d, J = 6.8 Hz) |
| 36 | 7.98 (1H, m), 7.47 (1H, t, J = 8.9 Hz), 3.99 (3H, s), 3.01 (2H, q, J = 7.4 Hz), 2.35 (3H, s), 1.38 (3H, t, J = 7.4 Hz) |
| 37 | 7.98 (1H, dd, J1 = 9.1 Hz, J2 = 5.2 Hz), 7.47 (1H, t, J = 8.9 Hz), 4.33 (2H, t, J = 6.6 Hz), 3.00 (2H, q, J = 7.5 Hz), 2.34 (3H, s), 1.77 (2H, m), 1.48 (2H, m), 1.37 (3H, t, J = 7.5 Hz), 0.98 (3H, t, J = 7.3 Hz) |
| 38 | 7.98 (1H, dd, J1 = 9.3 Hz, J2 = 5.2 Hz), 7.46 (1H, t, J = 9.0 Hz), 4.39 (2H, q, J = 7.1 Hz), 3.00 (2H, q, J = 7.5 Hz), 2.34 (3H, s), 1.43 (3H, t, J = 7.1 Hz), 1.37 (3H, t, J = 7.4 Hz) |
| 39 | 7.54 (1H, m), 7.26 (1H, dd, J1 = 18.0 Hz, J2 = 9.7 Hz), 3.11 (2H, m), 2.50 (3H, s), 2.37 (3H, s), 1.40 (3H, t, J = 7.6 Hz) |
| 40 | 7.86 (1H, m), 7.51 (1H, dd, J1 = 17.8 Hz, J2 = 9.8 Hz), 7.17 (1H, dd, J1 = 13.9 Hz, J2 = 6.1 Hz), 5.13 (1H, dd, J1 = 13.7 Hz, J2 = 2.3 Hz), 4.76 (1H, dd, J1 = 12.2 Hz, J2 = 2.5 Hz), 3.02 (2H, q, J = 7.5), 2.39 (3H, s), 1.39 (3H, t, J = 7.6) |
| 41 | 7.86 (1H, m), 7.51 (1H, dd, J1 = 17.8 Hz, J2 = 9.8 Hz), 4.92 (2H, d, J = 2.4 Hz), 3.02 (2H, q, J = 7.6 Hz), 2.63 (1H, t, J = 2.5 Hz), 2.38 (3H, s), 1.38 (3H, t, J = 7.6 Hz) |
| 42 | 7.86 (1H, m), 7.51 (1H, dd, J1 = 17.8 Hz, J2 = 9.8 Hz), 5.88 (2H, s), 3.02 (2H, q, J = 7.5 Hz), 2.37 (3H, s), 1.39 (3H, t, J = 7.6 Hz) |
| 43 | 7.85 (1H, m), 7.50 (1H, dd, J1 = 18.0 Hz, J2 = 9.8 Hz), 4.47 (2H, s), 3.01 (2H, q, J = 7.5 Hz), 2.34 (3H, s), 1.38 (3H, t, J = 7.5 Hz) |
| 44 | 7.81 (1H, m), 7.48 (1H, dd, J1 = 17.8 Hz, J2 = 9.5 Hz), 2.71 (3H, s), 2.46 (3H, s), 2.29 (3H, s) |
| 45 | 7.81 (1H, m), 7.49 (1H, dd, J1 = 17.8 Hz, J2 = 9.5 Hz), 4.40 (2H, q, J = 7.2 Hz), 2.72 (3H, s), 2.35 (3H, s), 1.44 (3H, t, J = 7.2 Hz) |
| 46 | 7.91 (1H, m), 7.53 (1H, m), 2.76 (3H, brs), 2.50 (3H, s), 2.34 (3H, s) |
| 47 | 7.48 (1H, m), 7.49 (1H, dd, J1 = 18.0 Hz, J2 = 9.8 Hz), 6.73 (1H, dd, J1 = 17.3 Hz, J2 = 1.3 Hz), 6.46 (1H, dd, J1 = 9.3 Hz, J2 = 10.5 Hz), 6.16 (1H, dd, J1 = 10.6 Hz, J2 = 1.1 Hz), 3.02 (2H, q, J = 7.5 Hz), 2.31 (3H, s), 1.38 (3H, t, J = 7.6 Hz) |
| 48 | 7.82 (1H, m), 7.48 (1H, dd, J1 = 18.0 Hz, J2 = 9.8 Hz), 3.00 (3H, m), 2.29 (3H, s), 1.43 (6H, d, J = 7.1 Hz), 1.37 (3H, t, J = 7.6 Hz) |
| 49 | 7.81 (1H, m), 7.49 (1H, dd, J1 = 18.0 Hz, J2 = 9.5 Hz), 2.71 (3H, s), 2.26 (3H, s), 2.14 (1H, m), 2.04 (1H, m), 1.69 (3H, brd) |
| 50 | 7.86 (1H, m), 7.52 (1H, m), 4.92 (2H, d, J = 2.4 Hz), 2.74 (3H, s), 2.63 (1H, t, J = 2.5 Hz), 2.36 (3H, s) |
| 51 | 7.82 (1H, m), 7.51 (1H, dd, J1 = 17.8 Hz, J2 = 9.5 Hz), 7.17 (1H, dd, J1 = 13.9 Hz, J2 = 6.1 Hz), 5.13 (1H, dd, J1 = 13.8 Hz, J2 = 2.4 Hz), 4.76 (1H, dd, J1 = 6.1 Hz, J2 = 2.2 Hz), 2.72 (3H, s), 2.36 (3H, s) |
| 52 | 7.80 (1H, m), 7.49 (1H, dd, J1 = 17.8 Hz, J2 = 9.7 Hz), 4.44 (2H, t, J = 7.0 Hz), 2.71 (2H, m), 2.71 (3H, s), 2.35 (3H, s), 2.07 (1H, t, J = 2.5 Hz) |
| 53 | 7.81 (1H, m), 7.71 (1H, d, J = 13.6 Hz), 7.49 (1H, dd, J1 = 17.9 Hz, J2 = 9.7 Hz), 6.57 (1H, d, J = 13.7 Hz), 2.87 (3H, s), 2.28 (3H, s) |
| 54 | 7.80 (1H, m), 7.48 (1H, dd, J1 = 17.8 Hz, J2 = 9.5 Hz), 7.04 (1H, d, J = 8.3 Hz), 6.59 (1H, d, J = 8.3 Hz), 2.88 (3H, s), 2.28 (3H, s) |
| 55 | 7.81 (1H, m), 7.48 (1H, dd, J1 = 17.8 Hz, J2 = 9.5 Hz), 2.99 (4H, m), 2.33 (3H, s), 1.77 (2H, m), 1.37 (3H, t, J = 7.6 Hz), 1.05 (3H, t, J = 7.3 Hz) |
| 56 | 7.83 (1H, m), 7.49 (1H, dd, J1 = 17.7 Hz, J2 = 9.7 Hz), 4.44 (2H, t, J = 6.9 Hz), 3.00 (2H, q, J = 7.5 Hz), 2.71 (2H, m), 2.06 (1H, t, J = 2.7 Hz), 1.57 (3H, s), 1.38 (3H, t, J = 7.5 Hz) |
| 57 | 7.80 (1H, m), 7.49 (1H, dd, J1 = 17.8 Hz, J2 = 9.7 Hz), 6.03 (1H, m), 5.47 (1H, dd, J1 = 17.2 Hz, J2 = 1.4 Hz), 5.38 (1H, dd, J1 = 10.3 Hz, J2 = 1.1 Hz), 4.82 (2H, d, J = 5.8 Hz), 2.71 (3H, s), 2.35 (3H, s) |
| 58 | 7.79 (1H, m), 7.49 (1H, dd, J1 = 17.8 Hz, J2 = 9.5 Hz), 2.91 (2H, t, J = 7.6 Hz), 2.70 (3H, s), 2.41 (2H, dt, J1 = 6.8 Hz, J2 = 2.7 Hz), 2.28 (3H, s), 2.05 (3H, m) |
| 59 | 7.80 (1H, m), 7.48 (1H, dd, J1 = 17.8 Hz, J2 = 9.5 Hz), 3.00 (2H, t, J = 7.3 Hz), 2.71 (2H, m), 2.70 (3H, s), 2.30 (3H, s), 2.07 (1H, m) |
| 60 | 7.82 (1H, m), 7.50 (1H, m), 3.62 (2H, brs), 2.72 (3H, brs), 2.30 (3H, brs) |
| 61 | 8.18 (1H, dd, J1 = 9.2 Hz, J2 = 4.9 Hz), 7.43 (1H, dd, J1 = 13.0 Hz, J2 = 10.6 Hz), 2.71 (3H, s), 2.24 (3H, s), 2.05 (1H, m), 1.19 (2H, m), 1.11 (2H, m) |
| 62 | 8.20 (1H, dd, J1 = 9.3 Hz, J2 = 4.9 Hz), 7.45 (1H, dd, J1 = 13.2 Hz, J2 = 10.7 Hz), 3.96 (3H, s), 2.73 (3H, s), 2.32 (3H, s) |
| 63 | 8.33 (1H, d, J = 6.6 Hz), 7.46 (1H, d, J = 10.8 Hz), 2.73 (3H, s), 2.28 (3H, s), 2.07 (1H, m), 1.30 (2H, m), 1.20 (2H, m) |
| 64 | 8.34 (1H, d, J = 6.6 Hz), 7.55 (1H, d, J = 10.7 Hz), 4.01 (3H, s), 2.74 (3H, s), 2.35 (3H, s) |
| 65 | 7.88 (1H, d, J = 9.0 Hz), 7.68 (1H, d, J = 9.0 Hz), 2.70 (3H, s), 2.48 (3H, s), 2.26 (3H, s) |
| 66 | 7.87 (1H, d, J = 9.0 Hz), 7.67 (1H, d, J = 9.0 Hz), 2.70 (3H, s), 2.26 (3H, s), 2.07 (1H, m), 1.27 (2H, m), 1.14 (2H, m) |
| 67 | 7.88 (1H, d, J = 9.1 Hz), 7.69 (1H, d, J = 9.1 Hz), 3.99 (3H, s), 2.71 (3H, s), 2.33 (3H, s) |
| 68 | 8.13 (1H, s), 7.78 (1H, s), 2.70 (3H, s), 2.52 (3H, s), 2.24 (3H, s) |
| 69 | 8.12 (1H, s), 7.81 (1H, s), 2.70 (3H, s), 2.24 (3H, s), 2.08 (1H, m), 1.30 (2H, m), 1.20 (1H, m) |
| 70 | 8.14 (1H, s), 7.90 (1H, s), 4.01 (3H, s), 2.71 (3H, s), 2.30 (3H, s) |
| 71 | 7.83 (1H, m), 7.48 (1H, dd, J1 = 18.0 Hz, J2 = 9.5 Hz), 4.41 (2H, t, J = 6.8 Hz), 3.00 (2H, q, J = 7.6 Hz), 2.37 (3H, s), 1.69 (2H, q, J = 6.9 Hz), 1.38 (3H, t, J = 7.5), 0.80 (1H, m), 0.52 (2H, m), 0.15 (2H, m) |
| 72 | 7.84 (1H, m), 7.50 (1H, dd, J1 = 18.0 Hz, J2 = 9.5 Hz), 4.56 (2H, t, J = 6.4 Hz), 3.01 (2H, q, J = 7.5 Hz), 2.65 (2H, m), 2.36 (3H, s), 1.38 (3H, t, J = 7.6 Hz) |
| 73 | 7.83 (1H, m), 7.48 (1H, dd, J1 = 18.0 Hz, J2 = 9.5 Hz), 4.39 (2H, t, J = 7.0 Hz), 3.00 (2H, q, J = 7.5 Hz), 2.64 (2H, m), 2.37 (3H, s), 1.80 (3H, t, J = 2.6 Hz), 1.38 (3H, t, J = 7.6 Hz) |
| 74 | 7.80 (1H, m), 7.49 (1H, dd, J1 = 18.0 Hz, J2 = 9.7 Hz), 4.41 (2H, t, J = 6.7 Hz), 2.71 (3H, s), 2.35 (3H, s), 1.69 (2H, q, J = 6.8 Hz), 0.80 (1H, m), 0.52 (2H, m), 0.41 (2H, m) |
| 75 | 7.81 (1H, m), 7.50 (1H, dd, J1 = 18.0 Hz, J2 = 9.5 Hz), 4.56 (2H, t, J = 6.4 Hz), 2.71 (3H, s), 2.65 (2H, m), 2.34 (3H, s) |
| 76 | 7.80 (1H, m), 7.49 (1H, dd, J1 = 18.0 Hz, J2 = 9.7 Hz), 4.39 (2H, t, J = 7.1 Hz), 2.71 (3H, s), 2.64 (2H, m), 2.35 (3H, s), 1.81 (3H, t, J = 2.6 Hz) |
| 77 | 7.76 (1H, dd, J1 = 11.2 Hz, J2 = 7.5 Hz), 7.53 (1H, dd, J1 = 10.4 Hz, J2 = 8.3 Hz), 4.41 (2H, t, J = 6.7 Hz), 2.70 (3H, s), 2.30 (3H, s), 1.69 (2H, q, J = 6.8 Hz), 0.80 (1H, m), 0.53 (2H, m), 0.17 (2H, m) |
| 78 | 7.77 (1H, dd, J1 = 11.2 Hz, J2 = 7.6 Hz), 7.49 (1H, dd, J1 = 10.5 Hz, J2 = 8.3 Hz), 4.57 (2H, t, J = 6.1 Hz), 2.71 (3H, s), 2.65 (2H, m), 2.29 (3H, s) |
| 79 | 7.76 (1H, dd, J1 = 11.2 Hz, J2 = 7.5 Hz), 7.55 (1H, dd, J1 = 10.5 Hz, J2 = 8.3 Hz), 4.39 (2H, t, J = 6.7 Hz), 2.70 (3H, s), 2.65 (2H, m), 2.31 (3H, s), 1.82 (3H, t, J = 2.4 Hz) |
| 80 | 7.85 (1H, m), 7.50 (1H, dd, J1 = 18.0 Hz, J2 = 9.5 Hz), 4.54 (2H, t, J = 6.4 Hz), 3.01 (2H, q, J = 7.5 Hz), 2.89 (2H, t, J = 6.5 Hz), 2.38 (3H, s), 1.38 (3H, t, J = 7.6 Hz) |
| 81 | 7.76 (1H, dd, J1 = 11.0 Hz, J2 = 7.5 Hz), 7.51 (1H, dd, J1 = 10.5 Hz, J2 = 8.3 Hz), 4.54 (2H, t, J = 6.3 Hz), 2.89 (2H, t, J = 6.3 Hz), 2.71 (3H, s), 2.31 (3H, s) |

| Preparation Example 1: WP (wettable powder) preparation | |
|---|---|
| Compound 2 | 10 wt% |
| Flumethrin | 15 wt% |
| Sodium diisobutylnaphthalenesulfonate | 1 wt% |
| Calcium n-dodecylbenzenesulfonate | 10 wt% |
| Alkylaryl polyglycol ether | 12 wt% |
| Sodium salt of naphthalenesulfonic acid formalin condensate | 3 wt% |
| Emulsion-type silicone | 1 wt% |
| Silicon dioxide | 3 wt% |
| Kaolin | 45 wt% |

| Preparation Example 2: Water-soluble concentrate preparation | |
|---|---|
| Compound 2 | 10 wt% |
| Flumethrin | 10 wt% |
| Polyoxyethylene lauryl ether | 3 wt% |
| Sodium dioctylsulfosuccinate | 3.5 wt% |
| Dimethyl sulfoxide | 37 wt% |
| 2-Propanol | 36.5 wt% |

| Preparation Example 3: Liquid preparation for spray | |
|---|---|
| Compound 2 | 1.0 wt% |
| Flumethrin | 1.0 wt% |
| Dimethyl sulfoxide | 10 wt% |
| 2-Propanol | 35 wt% |
| Acetone | 53 wt% |

| Preparation Example 4: Liquid preparation for percutaneous administration | |
|---|---|
| Compound 2 | 3 wt% |
| Flumethrin | 2 wt% |
| Hexylene glycol | 50 wt% |
| Isopropanol | 45 wt% |

| Preparation Example 5: Liquid preparation for percutaneous administration | |
|---|---|
| Compound No. 2 | 3 wt% |
| Flumethrin | 2 wt% |
| Propylene glycol monomethyl ether | 50 wt% |
| Dipropylene glycol | 45 wt% |

| Preparation Example 6: Liquid preparation for percutaneous (pour-on) administration | |
|---|---|
| Compound 2 | 1 wt% |
| Flumethrin | 1 wt% |
| Light liquid paraffin | 98 wt% |

| Preparation Example 7: Liquid preparation for percutaneous (pour-on) administration | |
|---|---|
| Compound 2 | 1 wt% |
| Flumethrin | 1 wt% |
| Light liquid paraffin | 58 wt% |
| Olive oil | 30 wt% |
| Medium chain triglyceride (ODO-H) | 9 wt% |
| Silicone oil (trade name: Shin-Etsu silicone, manufactured by Shin-Etsu Chemical Co., Ltd.) | 1 wt% |

### Test Example 1: Miticidal effect against Dermanyssus gallinae

The tip of a Pasteur pipette was sealed with Parafilm, and a sample compound diluted with acetone to a designated concentration was poured into the pipette from its top. The Parafilm in the tip of the pipette was removed one min after the pouring to discharge the test solution, and the pipette was air dried. Thereafter, an absorbent cotton was inserted into the upper end of the pipette, and 10 sucking adult mites of Dermanyssus gallinae were released in the pipette. The tip of the pipette was sealed with Hematoseal, and the pipette was then placed in a desiccator containing a saturated ammonium sulfate solution in its bottom and was stored under fully darkened conditions at 25°C. The mites were observed under a stereomicroscope 4 hr after and 24 hr after the treatment to judge whether the mites were dead or survived, followed by determination of the miticidal rate (%).

The results were as shown in Table A below.

**Table A:**

| Miticidal effect against Dermanyssus gallinae | | | |
|---|---|---|---|
| Test drug | Concentration of active ingredient, ppm | Miticidal rate 4 hr after treatment, % | Miticidal rate 24 hr after treatment, % |
| Compound 2 + flumethrin | 5 + 50 | 80 | 90 |
| Compound 2 + permethrin | 5 + 50 | 80 | 80 |
| Compound 2 + cyfluthrin | 5 + 50 | 80 | 80 |
| Compound 25 + flumethrin | 5 + 50 | 90 | 100 |
| Compound 25 + permethrin | 5 + 50 | 90 | 90 |
| Compound 25 + cyfluthrin | 5 + 50 | 90 | 90 |
| Compound 2 | 5 | 40 | 50 |
| Compound 25 | 5 | 70 | 80 |
| Flumethrin | 50 | 5 | 10 |
| Permethrin | 50 | 0 | 0 |
| Cyfluthrin | 50 | 0 | 0 |

## Claims

1. A mixture composition for controlling ectoparasites of mammals and birds, comprising as active ingredients:
(a) a compound of formula (I) or a salt thereof: 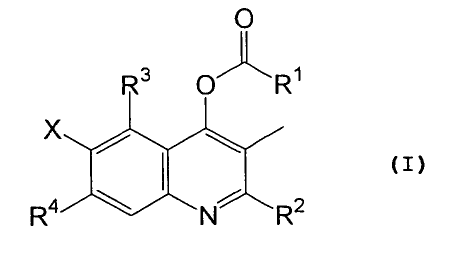 wherein
R¹ represents alkyl having 1 to 6 carbon atoms optionally substituted by a halogen atom or cyano; alkenyl having 2 to 6 carbon atoms optionally substituted by a halogen atom or cyano; alkynyl having 2 to 6 carbon atoms optionally substituted by a halogen atom or cyano; group -O-R⁵ wherein R⁵ represents alkyl having 1 to 6 carbon atoms optionally substituted by a halogen atom or cyano, alkenyl having 2 to 6 carbon atoms optionally substituted by a halogen atom or cyano, or alkynyl having 2 to 6 carbon atoms optionally substituted by a halogen atom or cyano; or group -S-R⁵ wherein R⁵ is as defined above,
R² represents alkyl having 1 to 4 carbon atoms optionally substituted by a halogen atom,
any one of R³ and R⁴ represents a hydrogen atom and the other represents a fluorine atom, a chlorine atom, a bromine atom, or trifluoromethyl, and
X represents a fluorine atom or a chlorine atom, and
(b) one or more compounds selected from the group consisting of pyrethroid insecticides.

2. The mixture composition according to claim 1, wherein the pyrethroid insecticide is selected from the group consisting of flumethrin, permethrin, and cyfluthrin.

3. The mixture composition according to claim 1 or 2, wherein the compound of formula (I) is represented by formula (Ia): 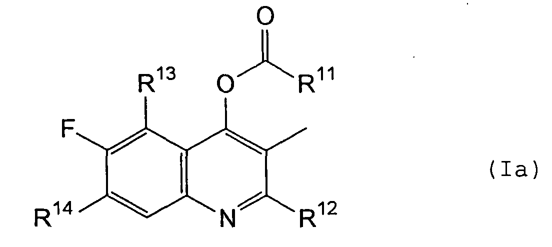 wherein
R¹¹ represents alkyl having 1 to 4 carbon atoms optionally substituted by a halogen atom or alkoxy having 1 to 4 carbon atoms optionally substituted by a halogen atom;
R¹² represents alkyl having 1 to 4 carbon atoms optionally substituted by a halogen atom; and
any one of R¹³ and R¹⁴ represents a hydrogen atom and the other represents a fluorine or chlorine atom.

4. The mixture composition according to any one of claims 1 to 3, wherein the compound of formula (I) is selected from the group consisting of:
2-ethyl-3-methyl-4-cyclopropanecarbonyloxy-5,6-difluoroquinoline (Compound 2);
2-ethyl-3-methyl-4-acetyloxy-5-chloro-6-fluoroquinoline (Compound 19);
2-ethyl-3-methyl-4-methoxycarbonyloxy-5,6-difluoroquinoline (Compound 24);
2,3-dimethyl-4-methoxycarbonyloxy-5,6-difluoroquinoline (Compound 25);
2-ethyl-3-methyl-4-methanethiolcarbonyloxy-5,6-difluoroquinoline (Compound 39);
2-ethyl-3-methyl-4-propargyloxycarbonyloxy-5,6-difluoroquinoline (Compound 41);
2,3-dimethyl-4-acetoxy-5,6-difluoroquinoline (Compound 44);
2,3-dimethyl-4-methanethiolcarbonyloxy-5,6-difluoroquinoline (Compound 46) ;
2,3-dimethyl-4-propargyloxycarbonyloxy-5,6-difluoroquinoline (Compound 50);
2,3-dimethyl-4-(3-butynyl)oxycarbonyloxy-5,6-difluoroquinoline (Compound 52);
2-ethyl-3-methyl-4-(3-butynyl)-oxycarbonyloxy-5,6-difluoroquinoline (Compound 56);
2,3-dimethyl-4-allyloxycarbonyloxy-5,6-difluoroquinoline (Compound 57);
2,3-dimethyl-4-aoetoxy-5,6-dichloroquinoline (Compound 65); and
2,3-dimethyl-4-methoxycarbonyloxy-5,6-dichloroquinoline (Compound 67).

5. The mixture composition according to any one of claims 1 to 4, wherein the ectoparasite is selected from the group consisting of Anoplura, Mallophaga, Siphonaptera, Mesostigmata, and Diptera.

6. A method for controlling an ectoparasite of mammals and birds, comprising the step of administering an effective amount of the mixture composition according to any one of claims 1 to 4 to a mammal or a bird of interest.

7. The method according to claim 6, wherein the ectoparasite is selected from the group consisting of Anoplura, Mallophaga, Siphonaptera, Mesostigmata, and Diptera.

8. A combination comprising:
(a) a compound of formula (I) or a salt thereof: 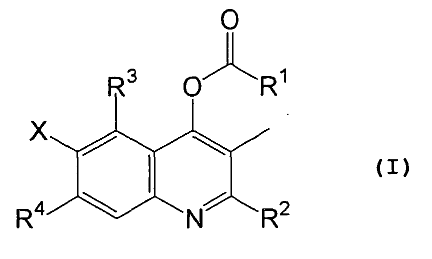 wherein
R¹ represents alkyl having 1 to 6 carbon atoms optionally substituted by a halogen atom or cyano; alkenyl having 2 to 6 carbon atoms optionally substituted by a halogen atom or cyano; alkynyl having 2 to 6 carbon atoms optionally substituted by a halogen atom or cyano; group -O-R⁵ wherein R⁵ represents alkyl having 1 to 6 carbon atoms optionally substituted by a halogen atom or cyano, alkenyl having 2 to 6 carbon atoms optionally substituted by a halogen atom or cyano, or alkynyl having 2 to 6 carbon atoms optionally substituted by a halogen atom or cyano; or group -S-R⁵ wherein R⁵ is as defined above,
R² represents alkyl having 1 to 4 carbon atoms optionally substituted by a halogen atom,
any one of R³ and R⁴ represents a hydrogen atom and the other represents a fluorine atom, a chlorine atom, a bromine atom, or trifluoromethyl, and
X represents a fluorine atom or a chlorine atom, and
(b) one or more compounds selected from the group consisting of pyrethroid insecticides.

9. Use of the combination according to claim 8, for the production of a mixture composition for controlling ectoparasites of mammals and birds.

10. The use according to claim 9, wherein the ectoparasite is selected from the group consisting of Anoplura, Mallophaga, Siphonaptera, Mesostigmata, and Diptera.

11. A method for controlling an ectoparasite of mammals and birds, comprising the step of administering simultaneously or separately to a mammal or a bird of interest:
(a) a compound of formula (I) or a salt thereof: 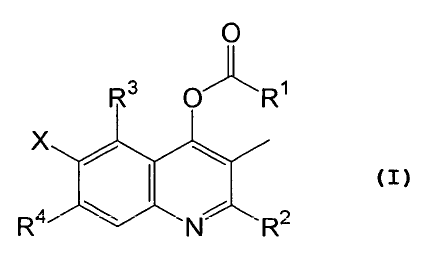 wherein
R¹ represents alkyl having 1 to 6 carbon atoms optionally substituted by a halogen atom or cyano; alkenyl having 2 to 6 carbon atoms optionally substituted by a halogen atom or cyano; alkynyl having 2 to 6 carbon atoms optionally substituted by a halogen atom or cyano; group -O-R⁵ wherein R⁵ represents alkyl having 1 to 6 carbon atoms optionally substituted by a halogen atom or cyano, alkenyl having 2 to 6 carbon atoms optionally substituted by a halogen atom or cyano, or alkynyl having 2 to 6 carbon atoms optionally substituted by a halogen atom or cyano; or group -S-R⁵ wherein R⁵ is as defined above,
R² represents alkyl having 1 to 4 carbon atoms optionally substituted by a halogen atom,
any one of R³ and R⁴ represents a hydrogen atom and the other represents a fluorine atom, a chlorine atom, a bromine atom, or trifluoromethyl, and
X represents a fluorine atom or a chlorine atom, and
(b) one or more compounds selected from the group consisting of pyrethroid insecticides.
